# EUROPEAN PATENT APPLICATION

(11) **EP 1 314 719 A1**
(43) Date of publication of application: **28.05.2003**
(21) Application number: 01956957.3
(22) Date of filing: 20.08.2001
(51) Int. Cl.: C07C 233/55, C07C 235/38, C07C 237/20, C07C 237/22, C07C 255/60, C07C 311/51, C07C 317/44, C07C 323/62, C07D 215/12, C07D 213/30, C07D 333/22, C07D 263/34, C07D 261/18, A61K 31/277, A61K 31/192, A61K 31/18, A61K 31/167, A61K 31/47, A61K 31/4406, A61K 31/381, A61K 31/421

(54) **BENZOIC ACID DERIVATIVES AND DRUGS CONTAINING THE SAME AS THE ACTIVE INGREDIENT**

(30) Priority: 01.09.2000 JP 2000264889
(71) Applicant: ONO PHARMACEUTICAL CO., LTD., Osaka-shi, Osaka 541-8526 (JP)
(72) Inventor: TANI, Kousuke, Ono Pharmaceutical Co., Ltd., Mishima-gun, Osaka 68-8585 (JP); ASADA, Masaki, Ono Pharmaceutical Co., Ltd., Mishima-gun, Osaka 618-8585 (JP); KOBAYASHI, Kaoru, Ono Pharmaceutical Co., Ltd., Mishima-gun, Osaka 618-8585 (JP); NARITA, Masami, Ono Pharmaceutical Co., Ltd., Mishima-gun, Osaka 618-8585 (JP); OGAWA, Mikio, Ono Pharmaceutical Co. Ltd., Mishima-gun, Osaka 618-8585 (JP)
(74) Representative: Henkel, Feiler, Hänzel
(86) International application number: JP0107105
(87) International publication number: WO02020462

(57) **Abstract**

A pharmaceutical composition, which comprises a benzoic acid derivative of formula (I) 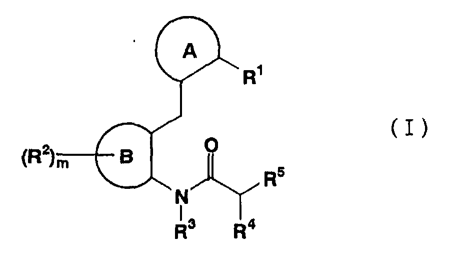 wherein R¹ is COOH, COOR⁶ etc.; A, B is carbocyclic ring or heterocyclic ring; R² is alkyl, alkenyl, alkynyl etc.; R⁴ is alkyl, cycloalkyl etc.; R⁵ is carbocyclic ring or heterocyclic ring;
or non-toxic salts thereof.

The compound of the formula (I) can bind to Prostaglandin E₂ receptors, especially, EP₃ receptor and/or EP₄ receptor and show the antagonizing activity, and useful for the prevention and/or treatment of disease, for example, pain, allergy, Alzheimer' s disease, cancer.

## Description

### Technical Field

The present invention relates to benzoic acid derivatives. More specifically, the present invention relates to a pharmaceutical agent comprising a benzoic acid derivative of formula (I) 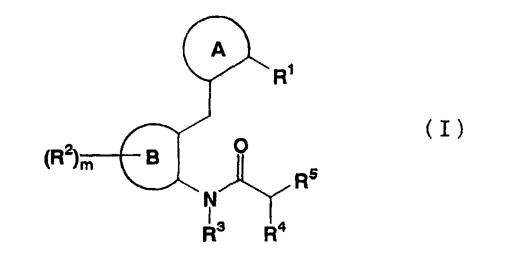 wherein all symbols are as hereinafter defined, or a non-toxic salt thereof as active ingredient, and a benzoic acid derivative of formula (I-A) 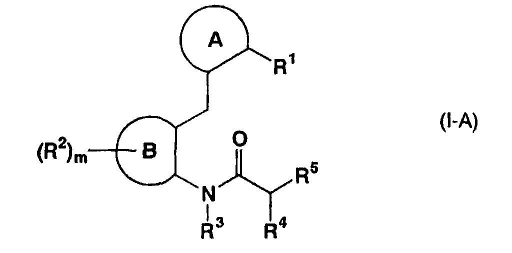 wherein all symbols are as hereinafter defined, or a non-toxic salt thereof, a process for the preparation thereof and a pharmaceutical agent comprising the same as active ingredient.

### Background

Prostaglandin E₂ (PGE₂) has been known as a metabolite in the arachidonic acid cascade. It has been known that PGE₂ possesses cytoprotective activity, uterine contractile activity, a pain-inducing effect, a promoting effect on digestive peristalsis, an awaking effect, a suppressive effect on gastric acid secretion, hypotensive activity, and diuretic activity.

In the recent study, it was found that PGE₂ receptor was divided into some subtypes, which possesses different physical roles from each other. At present, four receptor subtypes are known and they are called EP₁, EP₂, EP₃ and EP₄ respectively (J. Lipid Mediators Cell Signaling 12, 379-391 (1995)).

Among these subtypes, EP₃ receptor was believed to be involved in signal transduction of peripheral nerve, control of exothermal reaction in central nerve, formation of memory by expressing in cerebral neuron, vascularization, reabsorption of urine by expressing in renal tubular, uterine contraction, production of ACTH, platelet aggregation. Besides, it was expressed in vascular smooth muscle, heart and gastrointestinal tract also. EP₄ receptor was believed to be involved in suppression of TNF-α production and induction of IL-10 production.

So the compounds which can bind to EP₃ receptor and/or EP₄ receptor strongly and show the antagonizing activity, are useful for the prevention and/or treatment of diseases induced by excess activation of EP₃ receptor and/or EP₄ receptor, for example, pain such as cancerous pain, fractural pain, pain following surgical and dental procedures; allodynia, hyperalgesia, pruritus, urticaria, atopic dermatitis, contact dermatitis, allergic conjunctivitis, various symptoms by treating with dialysis, asthma, rhinitis, sneeze, urinary frequency, neurogenic bladder, urinary disturbance, ejaculatory failure, defervescence, systemic inflammatory response syndrome, learning disturbance, Alzheimer' s disease, cancer such as formulation of cancer, growth of cancer and metastasis of cancer; retinopathy, patch of red, scald, burn, burn by steroid, renal failure, nephropathy, acute nephritis, chronic nephritis, abnormal blood levels of electrolytes, threatened premature delivery, abortion threatened, hypermenorrhea, dysmenorrhea, uterine fibroids, premenstrual syndrome, reproductive disorder, stress, anxiety disorders, depression, psychosomatic disorder, mental disorder, thrombosis, embolism, transient ischemia attack, cerebral infarction, atheroma, organ transplant, myocardial infarction, cardiac failure, hypertension, arteriosclerosis, circulatory failure and circulatory failure induced ulcer, neuropathies, vascular dementia, edema, various arthritis, rheumatism, diarrhea, constipation, disorder of bilious excretion, ulcerative colitis, Crohn's disease and / or bone diseases such as osteoporosis, rheumatoid arthritis, osteoarthritis, abnormal bone formation; cancer such as formation of cancer, proliferation of cancer, metastasis of cancer to organs and to bones and hypercalcemia induced metastasis to bones of cancer; systemic granuloma, immunological diseases such as ALS, multiple sclerosis, Sjoegren's syndrome, systemic lupus erythematosus, AIDS; allergy such as conjunctivitis, rhinitis, contact dermatitis, psoriasis; atopic dermatitis, asthma, pyorrhea, gingivitis, periodontitis, neuronal cell death, Alzheimer' s disease's disease, pulmonary injury, hepatopathy, acute hepatopathy, nephritis, renal failure, myocardial ischemia, Kawasaki disease, scald, ulcerative colitis, Crohn's disease, multiple organ failure etc. Moreover, EP₄ is thought to be involved in sleeping disorder and platelet aggregation, so the compounds are considered to be useful.

### Disclosure of the Invention

The present inventors have energetically studied to find the compound which bind to PGE₂ receptor, EP₃ and / or EP₄ receptor specifically and show an inhibitory activity against it, to find out that the benzoic acid derivatives of formula (I) or formula (I-A) achieve the purpose and completed the present invention.

This invention was relates to
(1) a pharmaceutical composition having an activity of Prostaglandin E₂ receptor subtype EP₃ antagonist, which comprises a benzoic acid derivative of formula (I) 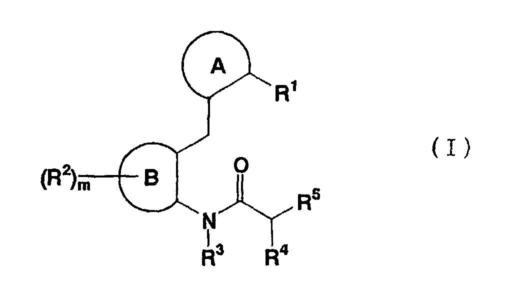 wherein R¹ is COOH, COOR⁶_{,} CH₂OH, CONHSO₂R⁷ or CONR⁸R⁹,
   R⁶ is C1-6 alkyl, (C1-4 alkylene)-R¹⁶,
   R⁷ is (1) C1-4 alkyl, or (2) substituted by 1-2 of substitutes selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituents or unsubstituted carbocyclic ring or heterocyclic ring,
   R⁸ and R⁹ each independently, is hydrogen or C1-4 alkyl,
   R¹⁶ is hydroxy, C1-4 alkoxy, COOH, C1-4 alkoxycarbonyl, CONR⁸R⁹,
   A is C5-6 mono-carbocyclic ring or 5-6 membered mono-heterocyclic ring containing at least one of nitrogen atom, oxygen atom and sulfur atom, the mono-carbocyclic ring or mono-heterocyclic ring may be substituted by 1-2 of substitutes selected from C 1-4 alkyl, C 1-4 alkoxy, halogen atom, CF₃, cyano and nitro,
   R² is C1-6 alkyl, C2-6 alkenyl, C2-6 alkynyl, C1-6 alkoxy, halogen atom, CF₃, cyano, hydroxy, nitro, NR¹⁰R¹¹, CONR¹⁰R¹¹, SO₂NR¹⁰R¹¹, or -S(O)ₓ-(C 1-4)alkyl,
   m is 0, 1 or 2, when m is 2, then two R² may be same or difference,
   R¹⁰ and R¹¹ each independently, hydrogen or C1-4 alkyl,
   x is 0, 1 or 2,
   B is C5-7 mono-carbocyclic ring or 5-7 membered mono-heterocyclic ring containing at least one of nitrogen atom, oxygen atom and sulfur atom,
   R³ is hydrogen or C1-4 alkyl,
   R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 aLkoxy(C1-4)alkoxy, phenyl and C3-6 cycloalkyl,
   R⁶ is substituted by 1-2 of R¹² or unsubstituted C5-10 mono- or bi-carbocyclic ring or 5-10 membered mono- or bi-heterocyclic ring containing at least one of nitrogen atom, oxygen atom and sulfur atom,
   R¹² is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{y}-G-(C1-8 alkylene)_{z}-R¹³, benzoyl or thiophenecarbonyl and two R¹² may be same or difference,
   y is 0 or 1,
   z is 0 or 1,
   R¹³ is phenyl or pyridyl,
   G is oxygen atom, S(O)ₜ or NR¹⁴,
   t is 0, 1 or 2,
   R¹⁴ is hydrogen, C1-4 alkyl or acetyl;
   or non-toxic salts thereof as active ingredients,
(2) a benzoic acid derivative of formula (I-A) 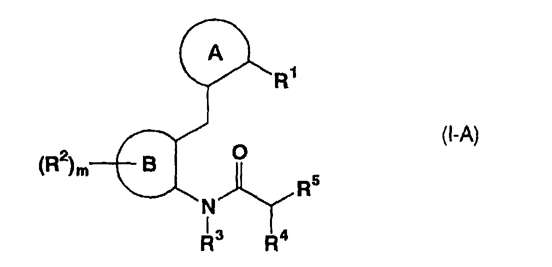 wherein R¹ is COOH, COOR⁶, CH₂OH, CONHSO₂R⁷ or CONR⁸R⁹,
   R⁶ is C 1-6 alkyl, (C 1-4 alkylene)-R¹⁶,
   R⁷ is (1) C1-4 alkyl, or (2) substituted by 1-2 of substitutes selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituents or unsubstituted carbocyclic ring or heterocyclic ring,
   R⁸ and R⁹ each independently, is hydrogen or C1-4 alkyl,
   R¹⁶ is hydroxy, C1-4 alkoxy, COOH, C1-4 alkoxycarbonyl, CONR⁸R⁹,
   A is C5-6 mono-carbocyclic ring or 5-6 membered mono-heterocyclic ring containing at least one of nitrogen atom, oxygen atom and sulfur atom, the mono-carbocyclic ring or mono-heterocyclic ring may be substituted by 1-2 of substitutes selected from C1-4 alkyl, C1-4 alkoxy, halogen atom, CF₃, cyano and nitro,
   R² is C1-6 alkyl, C2-6 alkenyl, C2-6 alkynyl, C1-6 alkoxy, halogen atom, CF₃, cyano, hydroxy, nitro, NR¹⁰R¹¹, CONR¹⁰R¹¹, SO₂NR¹⁰R¹¹, or -S(O)ₓ-(C 1-4)alkyl,
   m is 0, 1 or 2, when m is 2, then two R² may be same or difference,
   R¹⁰ and R¹¹ each independently, hydrogen or C1-4 alkyl,
   x is 0, 1 or 2,
   B is C5-7 mono-carbocyclic ring or 5-7 membered mono-heterocyclic ring containing at least one of nitrogen atom, oxygen atom and sulfur atom,
   R³ is hydrogen or C1-4 alkyl,
   R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
   R⁶ is substituted by 1-2 of R¹² or unsubstituted C5-10 mono- or bi-carbocyclic ring or 5-10 membered mono- or bi-heterocyclic ring containing at least one of nitrogen atom, oxygen atom and sulfur atom,
   R¹² is C1-6 alkyl, C1-6 alkoxy, halogen atom, CF₃, cyano, C1-4 alkoxy(Cl-4)alkyl, phenyl, phenyl(C1-6) alkyl, -(C1-4 alkylene)_{y}-G-(C1-8 alkylene)_{z}-R¹³, benzoyl or thiophenecarbonyl and two R¹² may be same or difference,
   y is 0 or 1,
   z is 0 or 1,
   R¹³ is phenyl or pyridine,
   G is oxygen atom, S(O)ₜ or NR¹⁴,
   t is 0, 1 or 2,
   R¹⁴ is hydrogen, C1-4 alkyl or acetyl,
   with the proviso, at least one of the R¹, R², R⁴ and R¹² is as follows,
   R¹ is COO-(C1-4 alkylene)-R¹⁶, CH₂OH or CONHSO₂R⁷,
   R² is C2-6 alkenyl, C2-6 alkynyl, hydroxy, NR¹⁰R¹¹, CONR¹⁰R¹¹,-SO₂NR¹⁰R¹¹, or -S(O)ₓ-(C1-4)alkyl,
   R⁴ is (1) C6-8 alkyl, (2) C2-8 alkenyl, (3) C2-8 alkynyl, (4) C3-6 cycloalkyl, (5) hydroxy, (6) C1-4 alkoxy(C1-4)alkoxy, or (7) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
   R¹² is -(C1-4 alkylene)-G-(C1-8 alkylene)-R¹³, -(C1-4 alkylene)-G₁-R¹³, -G₁-(C1-8 alkylene)-R¹³, benzoyl or thiophenecarbonyl,
   G¹ is oxygen atom, S(O)ₜ or NR¹⁴;
   or non-toxic salts,
(3) a process of the preparation thereof, and
(4) a pharmaceutical composition having an activity of Prostaglandin E₂ receptor subtype EP₄ antagonist comprising the same as active ingredient.

### Detailed description of the Invention

In the present invention, C1-4 alkyl is methyl, ethyl, propyl, butyl and isomers thereof.

In the present invention, C1-6 alkyl is methyl, ethyl, propyl, butyl, pentyl, hexyl and isomers thereof.

In the present invention, C1-8 alkyl is methyl, ethyl, propyl, butyl, pentyl, hexyl, heptyl, octyl and isomers thereof.

In the present invention, C2-6 alkenyl is ethenyl, propenyl, butenyl, pentenyl, hexenyl and isomers thereof.

In the present invention, C2-8 alkenyl is ethenyl, propenyl, butenyl, pentenyl, hexenyl, heptenyl, octenyl and isomers thereof.

In the present invention, C2-6 alkynyl is ethynyl, propynyl, butynyl, pentynyl, hexynyl and isomers thereof.

In the present invention, C2-8 alkynyl is ethynyl, propynyl, butynyl, pentynyl, hexynyl, heptynyl, octynyl and isomers thereof.

In the present invention, C1-4 alkoxy is methoxy, ethoxy, propoxy, butoxy and isomers thereof.

In the present invention, C 1-6 alkoxy is methoxy, ethoxy, propoxy, butoxy, pentyloxy, hexyloxy and isomers thereof.

In the present invention, C1-4 alkoxy(C1-4)alkyl is, for example, methoxymethyl, methoxyethyl, methoxypropyl, methoxybutyl, ethoxymethyl, ethoxyethyl, ethoxypropyl, ethoxybutyl, propoxymethyl butoxymethyl and isomers thereof.

In the present invention, C1-4 alkoxy(C1-4)alkoxy is, for example, methoxymethoxy, methoxyethoxy, methoxypropoxy, methoxybutoxy, ethoxymethoxy, ethoxyethoxy, ethoxypropoxy, ethoxybutoxy and isomers thereof.

In the present invention, C1-4 alkoxycarbonyl is methoxycarbonyl, ethoxycarbonyl, propoxycarbonyl, butoxycarbonyl and isomers thereof.

In the present invention, C1-4 alkylene is methylene, ethylene, trimethylene, tetramethylene and isomers thereof.

In the present invention, C1-8 alkylene is methylene, ethylene, trimethylene, tetramethylene, pentamethylene, hexamethylene, heptamethylene, octamethylene and isomers thereof.

In the present invention, C3-6 cycloalkyl is cyclopropyl, cyclobutyl, cyclopentyl, and cyclohexyl.

In the present invention, phenyl(C1-6)alkyl is, for example, benzyl, phenylethyl, phenylpropyl, phenylbutyl and isomers thereof.

In the present invention, halogen atom is fluoride, chloride, bromide and iodide.

In the present invention, C6-12 mono- or bi-carbocyclic ring is C6-12 unsaturated, or partially or fully saturated mono- or bi-carbocyclic ring, for example, cyclohexane, cycloheptane, cyclohexene, benzene, indene, naphthalene, indan, tetrahydronaphthalene.

In the present invention, 5-15 membered mono- or bi-heterocyclic ring containing at least one of hetero atom selected from nitrogen atom, oxygen atom and sulfur atom is 5-15 membered unsaturated, or partially or fully saturated mono- or bi-heterocyclic ring containing 1-4 of nitrogen atom(s), 1-2 of oxygen atom(s), 1 of sulfur atom, 1 of nitrogen atom and 1 of oxygen atom, or 1 of nitrogen atom and 1 of sulfur atom, for example, furan, thiophene, pyrrole, oxazole, isoxazole, thiazole, isothiazole, imidazole, pyrazole, tetrazole, pyridine, pyrimidine, pyrazine, benzofuran, benzothiophene, benzothiazole, indole, benzoxazole, benzimidazole, benzodioxane, thienopyridine, indoline, isoindoline, 1,3-dioxaindan, chroman, isochroman, quinoline, isoquinoline, quinazoline, quinoxaline.

In the present invention, C5-6 mono-carbocyclic ring is C5-6 unsaturated, partially or fully saturated mono-carbocyclic ring, for example, cyclopentane, cyclohexane, cyclopentene, cyclohexene, benzene.

In the present invention, 5-6 membered mono-heterocyclic ring containing at least one of hetero atom selected from nitrogen atom, oxygen atom and sulfur atom is, for example, 5-6 membered mono-heterocyclic ring containing 1-2 of nitrogen atom(s), 1 of oxygen atom, 1 of sulfur atom, 1 of nitrogen atom and 1 of oxygen atom, or 1 of nitrogen atom and 1 of sulfur atom, concretely, furan, thiophene, pyrrole, oxazole, isoxazole, thiazole, isothiazole, imidazole, pyridine, pyrimidine, pyrazine.

In the present invention, 5-6 membered mono-heterocyclic ring containing 1-2 of nitrogen atom(s), 1 of sulfur atom, 1 of nitrogen atom and 1 of oxygen atom is pyrrole, pyridine, pyrimidine, pyrazine, thiophene, oxazole, isoxazole.

In the present invention, C5-7 mono-carbocyclic ring is C5-7 unsaturated, partially or fully saturated mono-carbocyclic ring, for example, cyclopentane, cyclohexane, cycloheptane, cyclopentene, cyclohexene, benzene.

In the present invention, 5-7 membered mono-heterocyclic ring containing at least one of hetero atom selected from nitrogen atom, oxygen atom and sulfur atom is 5-7 membered mono-heterocyclic ring containing 1-2 of nitrogen atom(s), 1-2 of oxygen atom(s) and / or 1 of sulfur atom, for example, furan, thiophene, pyrrole, oxazole, isoxazole, thiazole, isothiazole, imidazole, pyridine, pyrimidine, pyrazine, azepine.

In the present invention, 5-6 membered mono-heterocyclic ring containing 1-2 of nitrogen atom(s), 1 of oxygen atom and / or 1 of sulfur atom, for example, furan, thiophene, pyrrole, oxazole, isoxazole, thiazole, isothiazole, imidazole, pyridine, pyrimidine, pyrazine.

In the present invention, C5-10 mono- or bi-carbocyclic ring is C5-10 unsaturated, partially or fully saturated mono- or bi-carbocyclic ring, for example, cyclopentane, cyclohexane, cycloheptane, benzene, indan, indene, naphthalene, and tetrahydronaphthalene.

In the present invention, 5-10 membered mono- or bi-heterocyclic ring containing at least one of hetero atom selected from nitrogen atom, oxygen atom and sulfur atom is 5-10 membered unsaturated, partially or fully saturated mono- or bi-heterocyclic ring containing 1-4 of nitrogen atom(s), 1-2 of oxygen atom(s), 1 of sulfur atom, 1 of nitrogen atom and 1 of oxygen atom or 1 of nitrogen atom and 1 of sulfur atom, for example, furan, thiophene, pyrrole, oxazole, isoxazole, thiazole, isothiazole, imidazole, pyrazole, tetrazole, pyridine, pyrimidine, pyrazine, benzofuran, benzothiophene, benzothiazole, indole, benzoxazole, benzimidazole, benzodioxane, thienopyridine, indoline, isoindoline, 1,3-dioxaindane, chroman, isochroman, quinoline, isoquinoline, quinazoline, quinoxaline.

In the present invention, 5-10 membered mono- or bi-heterocyclic ring containing 1-2 of nitrogen atom(s), 1-2 of oxygen atom(s) and / or 1 of sulfur atom is 5-10 membered unsaturated, partially or fully saturated mono- or bi-heterocyclic ring containing 1-2 of nitrogen atom(s), 1-2 of oxygen atom(s), 1 of sulfur atom, 1 of nitrogen atom and 1 of oxygen atom or 1 of nitrogen atom and 1 of sulfur atom, for example, furan, thiophene, pyrrole, oxazole, isoxazole, thiazole, isothiazole, imidazole, pyrazole, pyridine, pyrimidine, pyrazine, benzofuran, benzothiophene, benzothiazole, indole, benzoxazole, benzoimidazole, benzodioxane, indoline, isoindoline, 1,3-dioxaindane, chroman, isochroman, quinoline, isoquinoline, quinazoline, quinoxaline.

Unless otherwise specified, all isomers are included in the present invention. For example, alkyl, alkenyl, alkynyl and alkylene groups include straight-chain and also branched-chain ones. In addition, isomers in double bond, ring, fused ring (E-, Z-, cis-, trans-isomer), isomers generated from asymmetric carbon atom(s) (R-, S-, α-, β-isomer, enantiomer, diastereomer), optically active isomers having optical rotation (D-, L-, d-, 1-isomer), polar compounds separated by chromatography (more polar compound, less polar compound), equilibrium compounds, mixtures thereof at arbitrary ratios and racemic mixtures are included in the present invention.

More preferably compound of the present invention of formula (I) is the compound which is
R¹ is COOH, COOR⁶, CH₂OH, CONHSO₂R⁷ or CONR⁸R⁹,
R⁶ is C1-6 alkyl, (C1-4 alkylene)-R¹⁶,
R⁷ is (1) C1-4 alkyl, or (2) substituted by 1-2 of selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituted or unsubstituted carbocyclic ring or heterocyclic ring,
R⁸ and R⁹ each independently, is hydrogen or C1-4 alkyl,
R¹⁶ is hydroxy, C1-4 alkoxy, COOH, C1-4 alkoxycarbonyl, CONR⁸R⁹,
A is C5-6 mono-carbocyclic ring or 5-6 membered mono-heterocyclic ring containing 1-2 of nitrogen atom(s), one of sulfur atom, or one of nitrogen atom and one of oxygen atom,
m is 0 or 1,
R² is C1-6 alkyl, C2-6 alkenyl, C2-6 alkynyl, C1-6 alkoxy, halogen atom, CF₃, cyano, hydroxy, nitro or NR¹⁰R¹¹,
B ring is C5-6 mono-carbocyclic ring or 5-6 membered mono-heterocyclic ring containing 1-2 of nitrogen atom(s), 1 of oxygen atom and / or 1 of sulfur atom,
R³ is hydrogen or C1-4 alkyl,
R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
R⁵ is C5-10 mono- or bi-carbocyclic ring substituted by 1-2 of R¹² or unsubstituted, or 5-10 membered mono- or bi-heterocyclic ring containing 1-2 of nitrogen atom(s), 1-2 of oxygen atom(s) and / or 1 of sulfur atom,
R¹² is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{y}-G-(C1-8 alkylene)_{z}-R¹³, benzoyl or thiophenecarbonyl and two R¹² may be same or difference,
y is 0 or 1,
z is 0 or 1,
R¹³ is phenyl or pyridyl,
G is oxygen atom, S(O)ₜ or NR¹⁴,
t is 0, 1 or 2,
R¹⁴ is hydrogen, C1-4 alkyl or acetyl.

In the present compound of formula (I), especially preferably R¹ is COOH, COOR⁶, CH₂OH, CONHSO₂R⁷ or CONR⁸R⁹, in which a preferably R⁷ is (1) C1-4 alkyl, (2) substituted by 1-2 of substitutes selected from C1-4 alkyl, C1-4 alkoxy and halogen atom, or unsubstituted cyclohexane, cycloheptane, cyclohexane, benzene, indene, naphthalene, indan, tetrahydronaphthalene, furan, thiophene, pyrrole, oxazole, isoxazole, thiazole, isothiazole, imidazole, pyrazole, tetrazole, pyridine, pyrimidine, pyrazine, benzofuran, benzothiophene, benzothiazole, indole, benzoxazole, benzoimidazole, benzodioxane, thienopyridine, indoline, isoindoline, 1,3-dioxaindan, chroman, isochroman, quinoline, isoquinoline, quinoxaline or (3) C1-2 alkyl substituted by the above ring described in (2), especially preferably R⁷ is (1) C1-4 alkyl, (2) substituted by 1-2 of substitutes selected from C1-4 alkyl, C1-4 alkoxy and halogen atom, or unsubstituted benzene, thiophene, oxazole, isoxazole, thiazole, isothiazole, pyridine or (3) C1-2 alkyl substituted by the above ring described in (2), and the other symbols are as hereinbefore defined.

In the compound of formula (I), concrete A ring is cyclohexane, benzene, pyrrole, thiophene, pyridine, pyrimidine, pyrazine, oxazole and isoxazole, and preferably A ring is cyclohexane, benzene, thiophene, pyridine, oxazole and isoxazole.

In the compound of formula (I), concrete B ring is cyclopentane, cyclohexane, cycloheptane, cyclopentene, cyclohexene, benzene, furan, thiophene, pyrrole, oxazole, isoxazole, thiazole, isothiazole, imidazole, pyridine, pyrimidine, pyrazine, and azepine.

Preferably B ring is cyclopentane, cyclohexane, benzene, furan, thiophene, pyrrole, imidazole, pyridine, pyrimidine, pyrazine, especially preferably, cyclohexane, benzene, thiophene, pyridine.

In the present invention, concrete R⁵ is substituted by 1-2 of R¹², wherein R¹² is as hereinbefore defined; or unsubstituted cyclopentane, cyclohexane, cycloheptane, benzene, indan, indene, naphthalene, tetrahydronaphthalene, furan, thiophene, pyrrole, oxazole, isoxazole, thiazole, isothiazole, imidazole, pyrazole, tetrazole, pyridine, pyrimidine, pyrazine, benzofuran, benzothiophene, benzothiazole, indole, benzoxazole, benzoimidazole, benzodioxane, thienopyridine, indoline, isoindoline, 1,3-dioxaindan, chroman, isochroman, quinoline, isoquinoline, quinazoline, quinoxaline.

Preferably R⁵ is substituted by 1-2 of R¹², wherein R¹² is as hereinbefore defined; or unsubstituted cyclohexane, benzene, naphthalene, tetrahydronaphthalene, furan, thiophene, pyrrole, pyridine, pyrimidine, pyrazine, benzofuran, benzothiophene, indole, benzodioxane, quinoline, isoquinoline, quinazoline, quinoxaline, especially preferably, substituted by 1-2 of R¹², wherein R¹² is as hereinbefore defined; or unsubstituted benzene, naphthalene, tetrahydronaphthalene, benzofuran, benzothiophene, indole, benzodioxane, quinoline.

The concrete examples of the compounds of formula (1) are the following compounds and non-toxic salts thereof.
(1) 2-[4-Cyano-2-[4-methyl-2-(1-naphthyl)pentanoylamino]phenyl]methylbenzoic acid,
(2) 2-[2-[2-(4-Benzyloxyphenyl)propanoylamino]phenyl]methylbenzoic acid,
(3) 2-[2-[2-(3-Benzyloxyphenyl)propanoylamino]phenyl]methylbenzoic acid,
(4) 2-[2-[2-(1-Naphthyl)butanoylamino]phenyl]methylbenzoic acid,
(5) 2-[2-[2-(1-Naphthyl)pentanoylamino]phenyl]methylbenzoic acid,
(6) 2-[2-[3-Methyl-2-(1-naphthyl)butanoylamino]phenyl]methylbenzoic acid,
(7) 2-[2-[2-(1,1'-Biphenyl-2-yl)propanoylamino]phenyl]methylbenzoic acid,
(8) 2-[2-[2-(1,1'-Biphenyl-3-yl)propanoylamino]phenyl]methylbenzoic acid,
(9) 2-[2-[2-(4-Phenoxyphenyl)propanoylamino]phenyl]methylbenzoic acid,
(10) 2-[2-[2-[4-(2-Phenylethoxy)phenyl]propanoylamino]phenyl]methylbenzoic acid,
(11) 2-[2-[2-[4-(3- Phenylpropoxy)phenyl]propanoylamino]phenyl]methylbenzoic acid,
(12) 2-[2-[2-Methoxy-2-(1-naphthyl)acetylamino]phenyl]methylbenzoic acid,
(13) 2-[2-[2-(4-Isobutylphenyl)propanoylamino]phenyl]methylbenzoic acid,
(14) 2-[2-[4-Methyl-2-(1-naphthyl)pentanoylamino]phenyl]methylbenzoic acid,
(15) 2-[2-[2-(1-Naphthyl)hexanoylamino]phenyl]methylbenzoic acid,
(16) 2-[2-[2-[4-(4-Phenylbutoxy)phenyl]propanoylamino]phenyl]methylbenzoic acid,
(17) 2-[2-[2(R)-(1-Naphthyl)propanoylamino]phenyl]methylbenzoic acid,
(18) 2-[2-[2(S)-(1-Naphthyl)propanoylamino]phenyl]methylbenzoic acid,
(19) 2-[2-[2-Ethoxy-2-(1-naphthyl)acetylamino]phenyl]methylbenzoic acid,
(20) 2-[2-[2-(1-Naphthyl)heptanoylamino]phenyl]methylbenzoic acid,
(21) 2-[2-[4,4-Dimethyl-2-(1-naphthyl)pentanoylamino]phenyl]methylbenzoic acid,
(22) 2-[2-[2-[4-(3-Phenylpropyl)phenyl]propanoylamino]phenyl]methylbenzoic acid,
(23) 2-[2-[2-(Quinolin-5-yl)propanoylamino]phenyl]methylbenzoic acid,
(24) 2-[2-[2-[4-[2-(3-Pyridyl)ethoxy]phenyl]propanoylamino]phenyl]methylbenzoic acid,
(25) 2-[2-[2-[4-(2-Phenoxyethyl)phenyl]propanoylamino]phenyl]methylbenzoic acid,
(26) 2-[2-[4-Methoxy-2-(1-naphthyl)butanoylamino]phenyl]methylbenzoic acid,
(27) 2-[2-[5-Methyl-2-(1-naphthyl)hexanoylamino]phenyl]methylbenzoic acid,
(28) 5-[2-[2-(1-Naphthyl)propanoylamino]phenyl]methyloxazole-4-carboxylic acid,
(29) 2-[2-[2-[4-(2-Phenylethylthio)phenyl]propanoylamino]phenyl]methylbenzoic acid,
(30) 2-[2-[3-Methoxy-2-(1-naphthyl)propanoylamino]phenyl]methylbenzoic acid,
(31) 2-[4-Ethoxy-2-[4-methyl-2-(1-naphthyl)pentanoylamino]phenyl]methylbenzoic acid,
(32) 2-[4-Isopropyloxy-2-[4-methyl-2-(1-naphthyl)pentanoylamino]phenyl]methylbenzoic acid,
(33) 5-[2-[4-Methyl-2-(1-naphthyl)pentanoylamino]phenyl]methyloxazole-4-carboxylic acid,
(34) 2-[4-Methoxy-2-[4-methyl-2-(1-naphthyl)pentanoylamino]phenyl]methylbenzoic acid,
(35) 2-[2-[2-[4-(2-Thienyl)carbonylphenyl]propanoylamino]phenyl]methylbenzoic acid,
(36) 2-[2-[3-Cyclopropyl-2-(1-naphthyl)propanoylamino]phenyl]methylbenzoic acid,
(37) 2-[2-[2-Cyclopropyl-2-(1-naphthyl)acetylamino]phenyl]methylbenzoic acid,
(38) 2-[2-[2-(4-Benzoylphenyl)propanoylamino]phenyl]methylbenzoic acid,
(39) 2-[2-[3-Phenyl-2-(1-naphthyl)propanoylamino]phenyl]methylbenzoic acid,
(40) 2-[2-[2-Methoxymethoxy-2-(1-naphthyl)acetylamino]phenyl]methylbenzoic acid,
(41) 2-[2-[3-Cyclobutyl-2-(1-naphthyl)propanoylamino]phenyl]methylbenzoic acid,
(42) 2-[2-[2-(4-Benzyloxymethylphenyl)propanoylamino]phenyl]methylbenzoic acid,
(43) 2-[2-[2-[4-[N-Methyl-N-(2-phenylethyl)amino]phenyl]propanoylamino]phenyl]methylbenzoic acid,
(44) 2-[2-[2-[4-[N-Acetyl-N-(2-phenylethyl)amino]phenyl]propanoylamino]phenyl]methylbenzoic acid,
(45) 2-[2-[2-[4-[N-(2-Phenylethyl)amino]phenyl]propanoylamino]phenyl]methylbenzoic acid,
(46) 2-[2-[2-[4-(2-Phenylethylsulfinyl)phenyl]propanoylamino]phenyl]methylbenzoic acid,
(47) 2-[2-[2-[4-(2-Phenylethylsulfonyl)phenyl]propanoylamino]phenyl]methylbenzoic acid,
(48) 2-[2-[2-(1-Naphthyl)-4-pentenoylamino]phenyl]methylbenzoic acid,
(49) 2-[2-[2-(1-Naphthyl)-4-hexenoylamino]phenyl]methylbenzoic acid,
(50) 2-[2-[4-Ethyl-2-(1-naphthyl)hexanoylamino]phenyl]methylbenzoic acid,
(51) 2-[2-[2-(1-Naphthyl)-4-pentynoylamino]phenyl]methylbenzoic acid,
(52) 2-[4-Cyano-2-[3-cyclopropyl-2-(1-naphthyl)propanoylamino]phenyl]methylbenzoic acid,
(53) 2-[2-[5-Methyl-2-(1-naphthyl)-4-hexenoylamino]phenyl]methylbenzoic acid,
(54) N-[2-[4-Cyano-2-[4-methyl-2-(1-naphthyl)pentanoylamino]phenyl]metnylbenzoyl]-phenylsulfonamide,
(55) 2-[2-[2-Hydroxy-2-(1-naphthyl)acetylamino]phenyl]methylbenzoic acid,
(56) 2-[4-Hydroxy-2-[4-methyl-2-(1-naphthyl)pentanoylamino]phenyl]methylbenzoic acid,
(57) 2-[4-Dimethylamino-2-[4-methyl-2-(1-naphthyl)pentanoylamino]phenyl]methylbenzoic acid and
(58) 2-[2-[2-(1-Naphthyl)propanoylamino]phenyl]methylbenzylalcohol.

More preferably compound of the present invention of formula (I-A) is the compound which is
R¹ is COOH_{,} COOR⁶, CH₂OH, CONHSO₂R⁷ or CONR⁸R⁹,
R⁶ is C1-6 alkyl, (C1-4 alkylene)-R¹⁶,
R⁷ is (1) C1-4 alkyl, or (2) substituted by 1-2 of selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituted or unsubstituted carbocyclic ring or heterocyclic ring,
R⁸ and R⁹ each independently, is hydrogen or C1-4 alkyl,
R¹⁶ is hydroxy, C1-4 alkoxy, COOH, C1-4 alkoxycarbonyl, CONR⁸R⁹,
A is C5-6 mono-carbocyclic ring or 5-6 membered mono-heterocyclic ring containing 1-2 of nitrogen atom(s), one of sulfur atom, or one of nitrogen atom and one of oxygen atom,
m is 0 or 1,
R² is C1-6 alkyl, C2-6 alkenyl, C2-6 alkynyl, C1-6 alkoxy, halogen atom, CF₃, cyano, hydroxy, nitro, NR¹⁰R¹¹, CONR¹⁰R¹¹, SO₂NR¹⁰R¹¹, or -S(O)ₓ-(C1-4)alkyl,
B ring is C5-6 mono-carbocyclic ring or 5-6 membered mono-heterocyclic ring containing 1-2 of nitrogen atom(s), 1 of oxygen atom and / or 1 of sulfur atom,
R³ is hydrogen or C 1-4 alkyl,
R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
R⁵ is C5-10 mono- or bi-carbocyclic ring substituted by 1-2 of R¹² or unsubstituted, or 5-10 membered mono- or bi-heterocyclic ring containing 1-2 of nitrogen atom(s), 1-2 of oxygen atom(s) and / or 1 of sulfur atom,
R¹² is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{y}-G-(C1-8 alkylene)_{z}-R¹³, benzoyl or thiophenecarbonyl and two R¹² may be same or difference,
y is 0 or 1,
z is 0 or 1,
R¹³ is phenyl or pyridyl,
G is oxygen atom, S(O)ₜ or NR¹⁴,
t is 0, 1 or 2,
R¹⁴ is hydrogen, C1-4 alkyl or acetyl,
with the proviso, at least one of the R¹, R², R⁴ and R¹² is as follows,
R¹ is COO-(C1-4 alkylene)-R¹⁶_{,} CH₂OH or CONHSO₂R⁷,
R² is C2-6 alkenyl, C2-6 alkynyl, hydroxy or NR¹⁰R¹¹,
R⁴ is (1) C6-8 alkyl, (2) C2-8 alkenyl, (3) C2-8 alkynyl, (4) C3-6 cycloalkyl, (5) hydroxy, (6) C1-4 alkoxy(C1-4)alkoxy, or (7) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
R¹² is -(C1-4 alkylene)-G-(C1-8 alkylene)-R¹³, -(C1-4 alkylene)-G₁-R¹³,-NR¹⁴-R¹³, benzoyl or thiophenecarbonyl.

The concrete examples of the compounds of formula (I-A) are the following compounds and non-toxic salts thereof.
(1) 2-[2-[2-[4-(2-Thienyl)carbonylphenyl]propanoylamino]phenyl]methylbenzoic acid,
(2) 2-[2-[3-Cyclopropyl-2-(1-naphthyl)propanoylamino]phenyl]methylbenzoic acid,
(3) 2-[2-[2-Cyclopropyl-2-(1-naphthyl)acetylamino]phenyl]methylbenzoic acid,
(4) 2-[2-[2-(4-Benzoylphenyl)propanoylamino]phenyl]methylbenzoic acid,
(5) 2-[2-[3-Phenyl-2-(1-naphthyl)propanoylamino]phenyl]methylbenzoic acid,
(6) 2-[2-[2-Methoxymethoxy-2-(1-naphthyl)acetylamino]phenyl]methylbenzoic acid,
(7) 2-[2-[3-Cyclobutyl-2-(1-naphthyl)propanoylamino]phenyl]methylbenzoic acid,
(8) 2-[2-[2-(4-Benzyloxymethylphenyl)propanoylamino]phenyl]methylbenzoic acid,
(9) 2-[2-[2-[4-[N-Methyl-N-(2-phenylethyl)amino]phenyl]propanoylamino]phenyl]methylbenzoic acid,
(10) 2-[2-[2-[4-[N-Acetyl-N-(2-phenylethyl)amino]phenyl]propanoylamino]phenyl]methylbenzoic acid,
(11) 2-[2-(2-[4-[N-(2-Phenylethyl)amino]phenyl]propanoylamino]phenyl]methylbenzoic acid,
(12) 2-[2-[2-[4-(2-Phenylethylsulfinyl)phenyl]propanoylamino]phenyl]methylbenzoic acid,
(13) 2-[2-[2-[4-(2-Phenylethylsulfonyl)phenyl]propanoylamino]phenyl]methylbenzoic acid,
(14) 2-[2- [2-(1-Naphthyl)-4-pentenoylamino]phenyl]methylbenzoic acid,
(15) 2-[2-[2-(1-Naphthyl)-4-hexenoylamino]phenyl]methylbenzoic acid,
(16) 2-[2-[4-Ethyl-2-(1-naphthyl)hexanoylamino]phenyl]methylbenzoic acid,
(17) 2-[2-[2-(1-Naphthyl)-4-pentynoylamino]phenyl]methylbenzoic acid,
(18) 2-[4-Cyano-2-[3-cyclopropyl-2-(1-naphthyl)propanoylamino]phenyl]methylbenzoic acid,
(19) 2-[2-[5-Methyl-2-(1-naphthyl)-4-hexenoylamino]phenyl]methylbenzoic acid,
(20) N-[2-[4-Cyano-2-[4-methyl-2-(1-naphthyl)pentanoylamino]phenyl]metnylbenzoyl]-phenylsulfonamide,
(21) 2-[2-[2-Hydroxy-2-(1-naphthyl)acetylamino]phenyl]methylbenzoic acid,
(22) 2-[4-Hydroxy-2-[4-methyl-2-(1-naphthyl)pentanoylamino]phenyl]methylbenzoic acid,
(23) 2-[4-Dimethylamino-2-[4-methyl-2-(1-naphthyl)pentanoylamino]phenyl]methylbenzoic acid and
(24) 2-[2-[2-(1-Naphthyl)propanoylamino]phenyl]methylbenzylalcohol.

### [Salt]

The compound of the present invention of formula (I) and formula (I-A) may be converted into a corresponding salt by known methods. In the present invention, non-toxic salts are salts of alkali metals, salts of alkaline-earth metals, ammonium salts, organic amines, acid addition salts and hydrates. Non-toxic and water-soluble salts are preferable.

Appropriate salts are, salts of alkali metals such as potassium, sodium, etc.; salts of alkaline-earth metals such as calcium, magnesium, etc.; ammonium salts, pharmaceutically acceptable organic amines such as tetramethylammonium, triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, etc.

Appropriate acid addition salts are, salts of inorganic acids such as hydrochloride, hydrobromide, sulfate, phosphate, nitrate; salts of organic acids e.g. acetate, trifluoroacetate, lactate, tartrate, oxalate, fumarate, maleate, citrate, benzoate, methanesulphonate, ethanesulphonate, benzenesulphonate, toluenesulphonate, isethionate, glucuronate, gluconate.

The compounds of formulae (I) and formulae (I-A) and salts thereof may be converted into the corresponding hydrates by conventional means.

### Preparation of the compound of the present invention

The present compound of formula (I) and formula (I-A) may be prepared, for example, by the following method.
(1) In the compound of formula (I) and formula (I-A), wherein R¹ is COOH, that is the compound of formula (Ia) 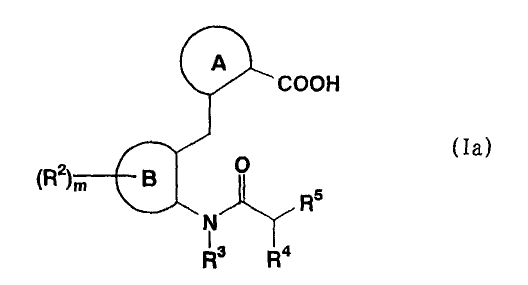 wherein all symbols are as hereinbefore defined;
   may be prepared by subjecting to hydrolysis under an alkaline conditions the compound of formula (Ib-1) 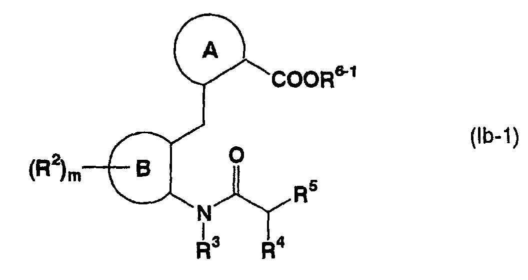 wherein R⁶⁻¹ is C1-6 alkyl and the other symbols are as hereinbefore defined.
   Hydrolysis under alkaline conditions is known, for example, it is carried out in a water-miscible organic solvent (e.g. methanol, ethanol, tetrahydrofuran, dioxane or a mixture thereof), using an aqueous solution of an alkali (e.g. sodium hydroxide, potassium hydroxide or potassium carbonate) at -10 - 90 °C.
(2) The compound of formula (Ib-1) may be prepared by amidation reaction the compound of formula (II) 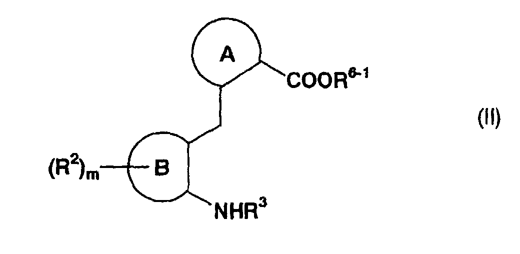 wherein all symbols are as hereinbefore defined; and the compound of formula (III) 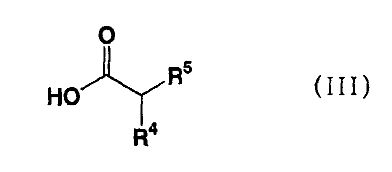 wherein all symbols are as hereinbefore defined.
   Amidation reaction is known, for example, it is carried out in an organic solvent (e.g. tetrahydrofuran, methylene chloride, chloroform, benzene, acetone, acetonitrile, diethyl ether or a mixture thereof), in the presence or absence of a tertiary amines (e.g. dimethylaminopyridine, pyridine, triethylamine), using a condensing agent (e.g. 1,3-dicyclohexylcarbodiimide (DCC), 1-ethyl-3-[3-(dimethylamino)propyl]carbodiimide (EDC), 2-chloro-1-methylpyridium iodide) or acyl halide (e.g. oxalyl chloride, thionyl chloride, phosphorus oxychloride) at 0 - 50 °C.
   In the compound of formula (Ib-1), wherein at least one of R² is hydroxy, that is the compound of formula (Ib-1a) 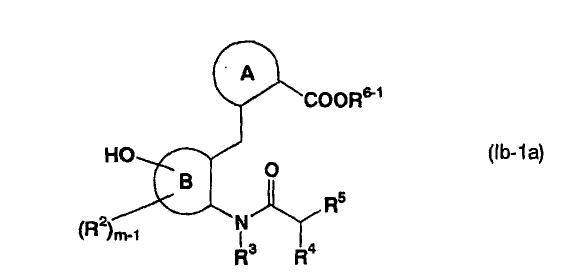 wherein m-1 is 0 or 1, the other symbols are as hereinbefore defined; may be prepared by deprotection reaction the compound of formula (IV) 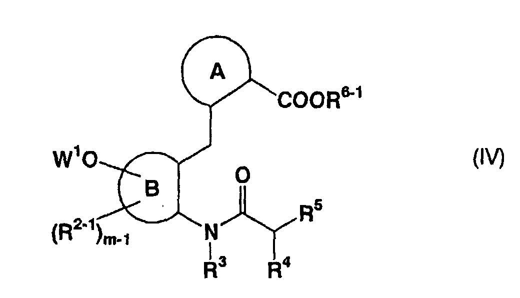 wherein W¹ is protective group of hydroxy, R²⁻¹ is the same meaning as R², with the proviso that, when it represents hydroxy, then the hydroxy is protected, the other symbols are as hereinbefore defined.
   Deprotection reaction is known, for example, it is carried out in organic solvent (e.g. methanol, ethanol, tetrahydrofuran, dioxane), using an acid (e.g. hydrochloric acid, sulfuric acid, p-toluenesulfonic acid) at 0-50 °C.
   In the compound of formula (Ib-1), wherein at least one of R² is NR¹⁰R¹¹, that is the compound of formula (Ib-1b) 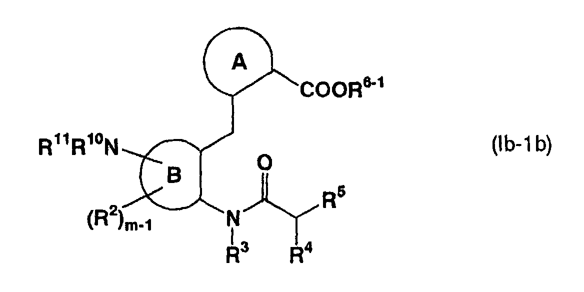 wherein all symbols are as hereinbefore defined;
   may be prepared by subjecting to N-alkylation reaction followed by deprotection or only deprotection the compound of formula (V) 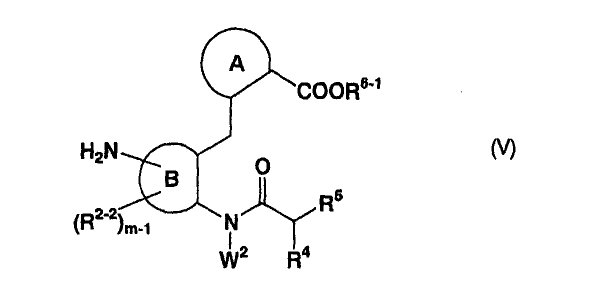 wherein W² is protective group of amino, R²⁻² is the same meaning as R², with the proviso that, when it represents NR¹⁰R¹¹, then it is NH₂, the other symbols are as hereinbefore defined.
   Alkylation reaction is known, for example, it is carried out in organic solvent (e.g. acetonitrile, THF, ethanol, methanol, dioxane), in the presence or absence of an acetic acid, using a formaldehyde and a sodium cyanoborohydride at 0 - 50 °C.
   Deprotection reaction is known, for example, it is carried out in organic solvent (e.g. methanol, ethanol, tetrahydrofuran, dioxane), using an acid (e.g. hydrochloric acid, sulfuric acid, p-toluenesulfonic acid) at 0-50 °C.
(3) In the compound of formula (I) or formula (I-A), wherein R¹ is CONHSO₂R⁷ and CONR⁸R⁹, that is the compound of formula (Ic) 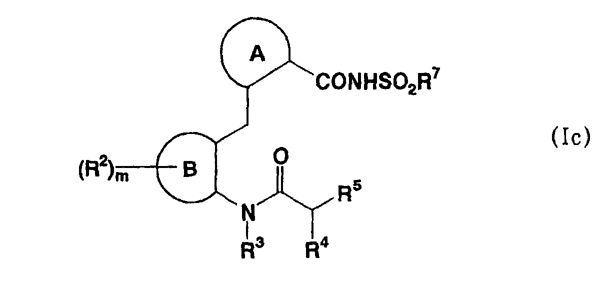 wherein all symbols are as hereinbefore defined; and the compound of formula (Id) 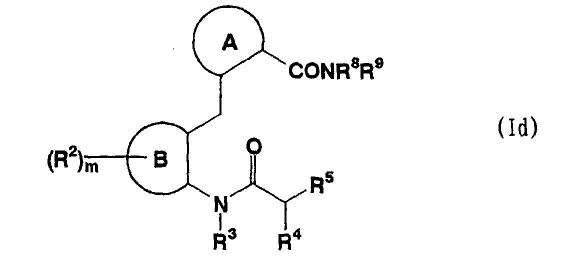 wherein all symbols are same as hereinbefore defined;
   may be prepared by subjecting to amidation reaction the compound of formula (Ia) and the compound of formula (VI-1)

   NH₂SO₂R⁷ (VI-1)

   wherein all symbols are as hereinbefore defined; or the compound of formula (VI-2)

   NHR⁸R⁹ (VI-2)

   wherein all symbols are as hereinbefore defined.
   Amidation reaction is carried out by the above method.
(4) In the compound of formula (I) or formula (I-A), wherein R¹ is CH₂OH, that is the compound of formula (Ie) 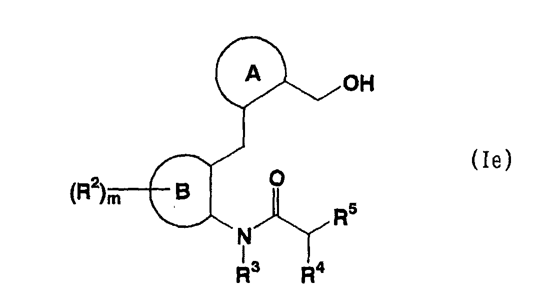 wherein all symbols are as hereinbefore defined;
   may be prepared by deprotection reaction the compound of formula (VII) 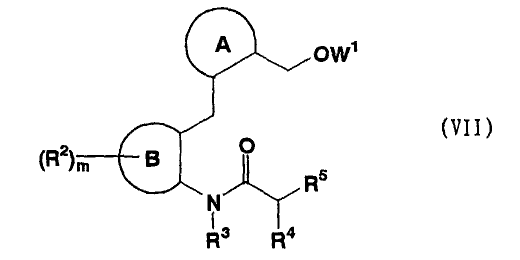 wherein all symbols are as hereinbefore defined.
   Deprotection reaction is known, for example, it is carried out in organic solvent (e.g. tetrahydrofuran, acetonitrile, methylene chloride), using a fluorinated compound (e.g. tetrabutylammonium fluoride) at 0 - 50 °C.
(5) In the compound of formula (I) or formula (I-A), wherein R¹ is COOR⁶⁻², in which R^{6·2} is -(C1-4 alkylene)-R¹⁶; that is the compound of formula (Ib-2) 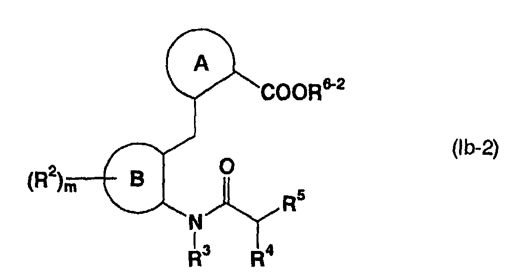 wherein all symbols are as hereinbefore defined;
   may be prepared by reacting the compound of formula (Ia) with the compound of formula (VIII)

   X-(C1-4 alkylene)-R¹⁶ (VIII)

   wherein X is halogen atom and the other symbols are as hereinbefore defined.

This reaction is known, for example, it is carried out in an organic solvent (e.g. dimethylformamide, tetrahydrofuran, acetone, acetonitrile), using potassium carbonate, sodium carbonate or sodium hydride, at 0 - 50 °C.

The compounds of formula (II), (III), (IV), (V), (VI-1), (VI-2), (VII) and (VIII) may be known per se, or may be prepared by known methods with ease. For example, the compounds of formula (II), (IV), (V) and (VII) may be prepared according to the following reaction schemes A, B-1, B-2, C-1, C-2 and D.

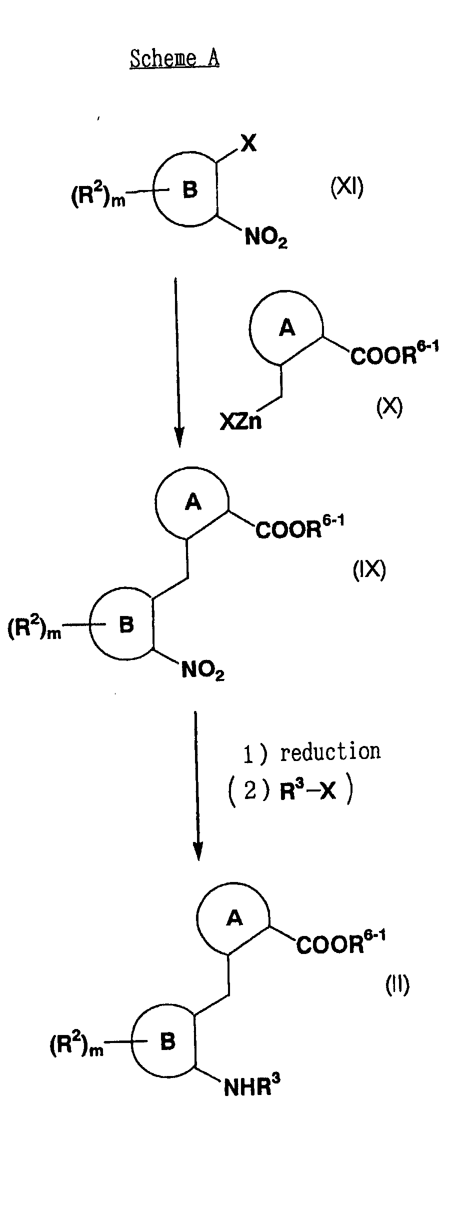

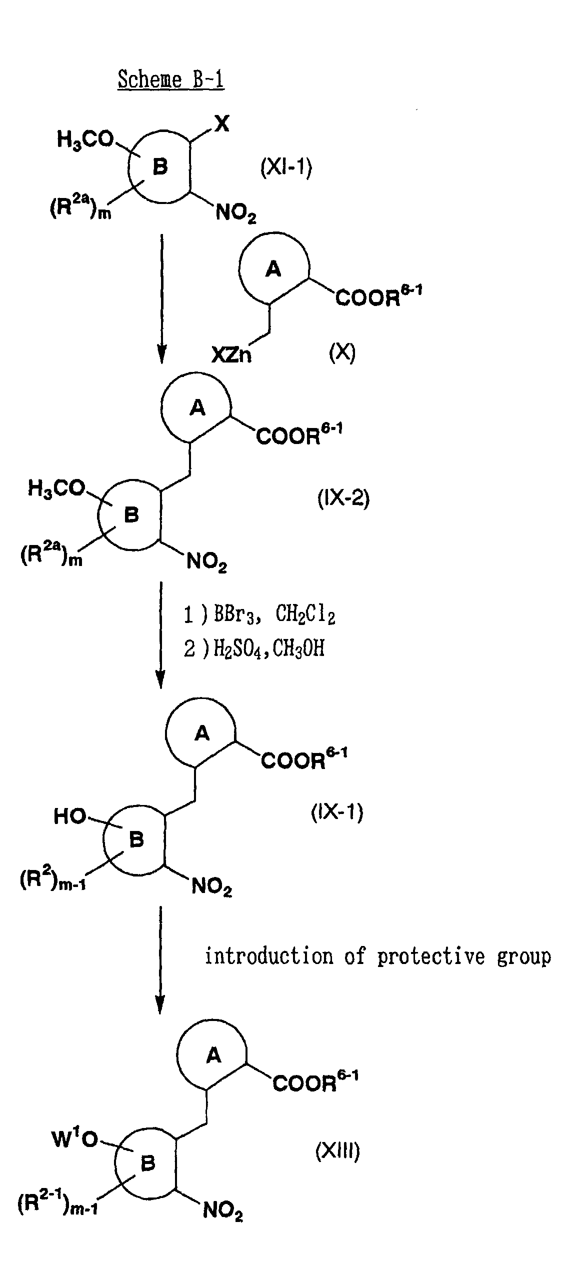

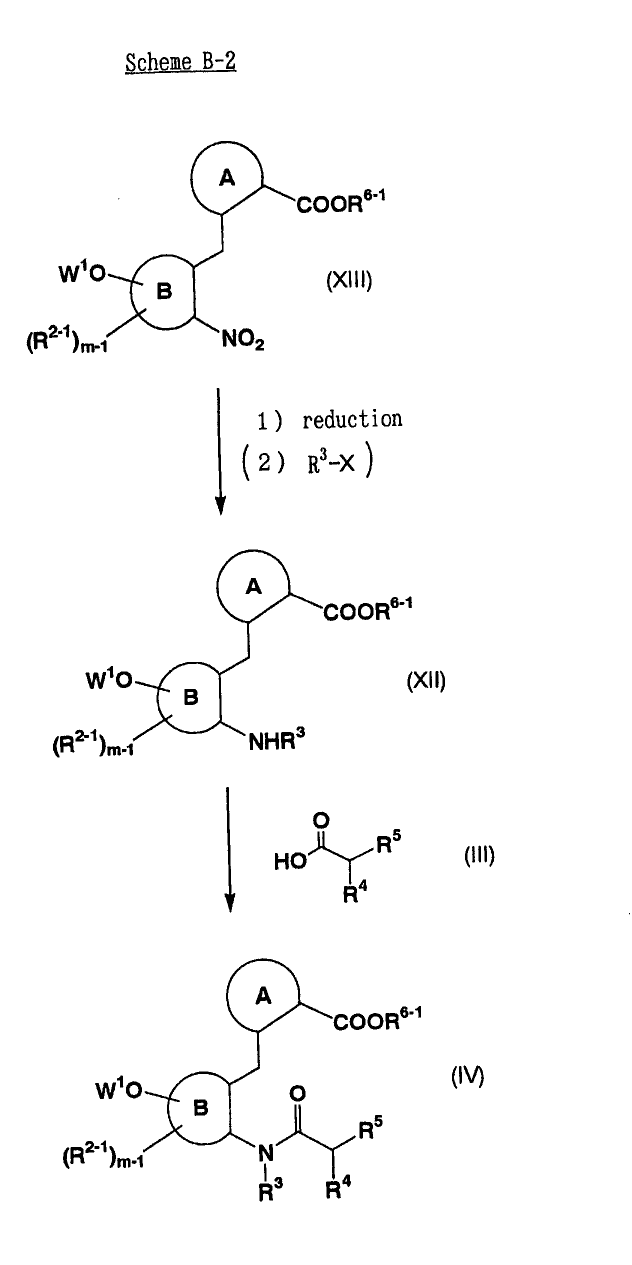

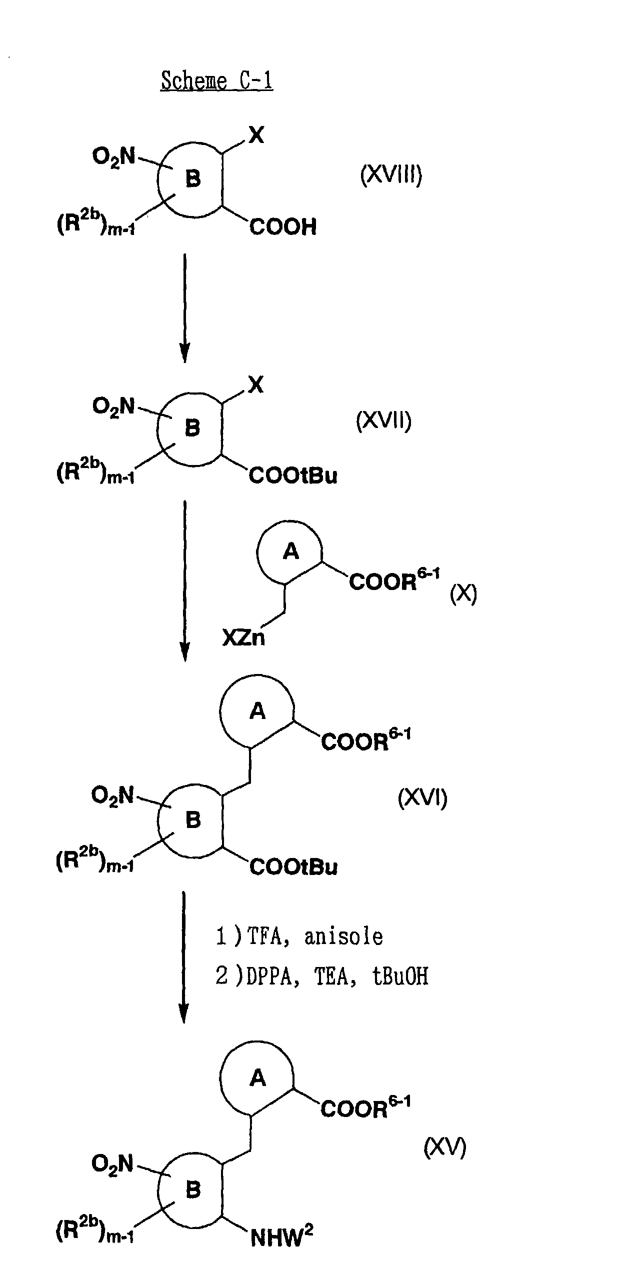

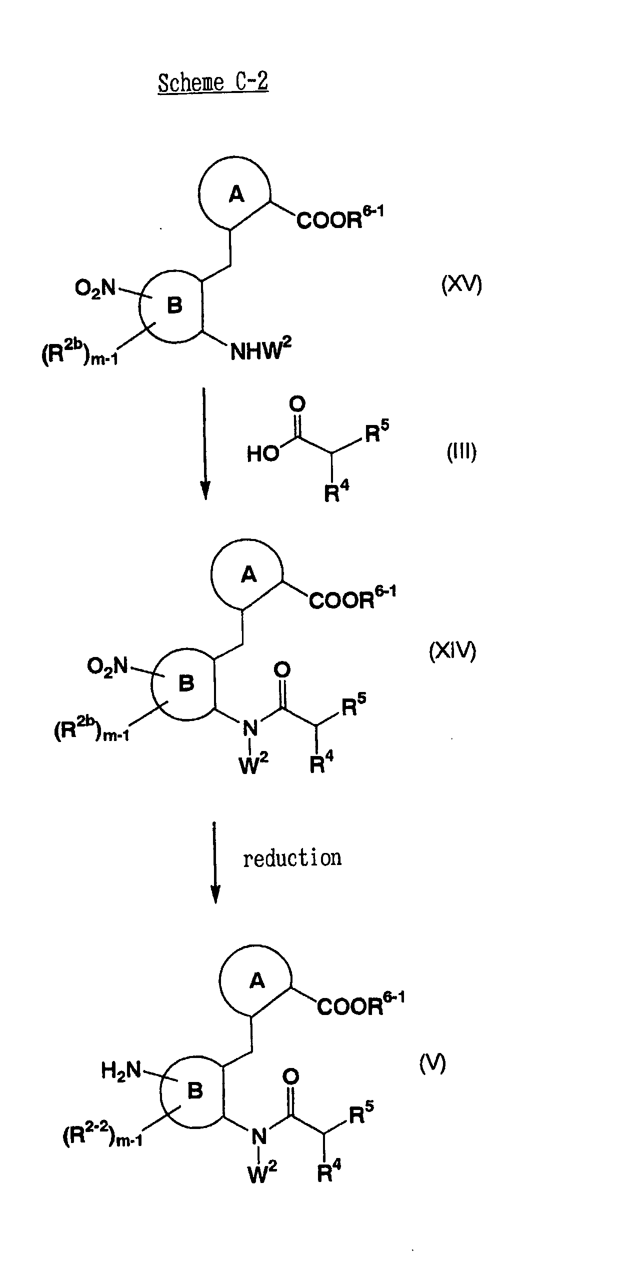

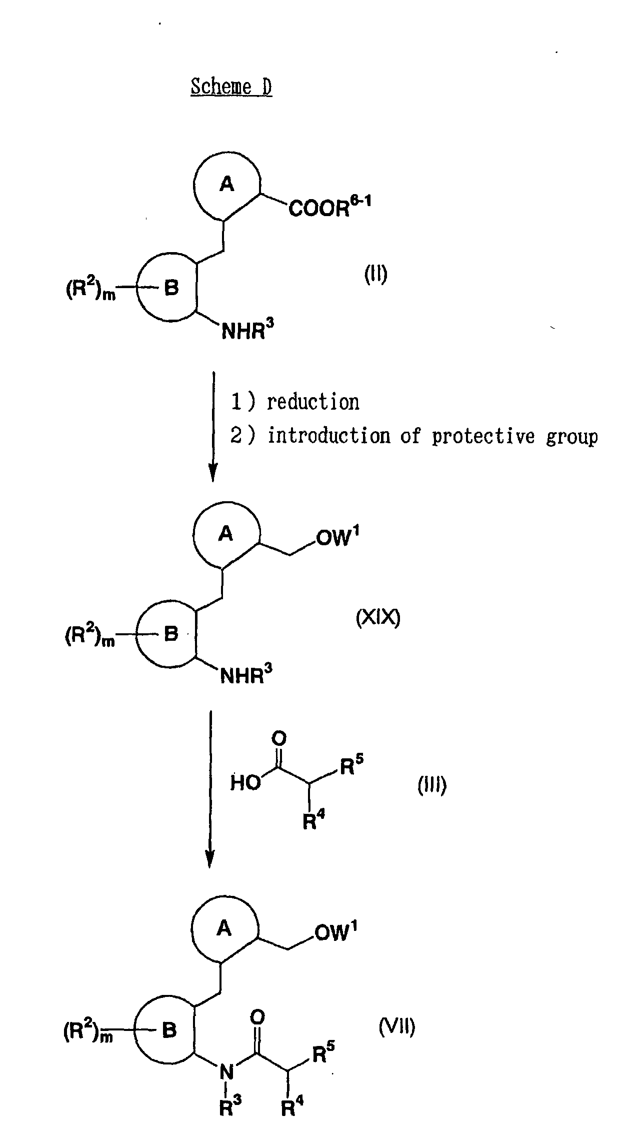

In above schemes
R^{2a} is the same meaning as R², with the proviso, R² is not hydroxy,
R^{2b} is the same meaning as R², with the proviso, R² is not NR¹⁰R¹¹, tBu is t-butyl,
DPPA is diphenylphosphoryl azide,
TEA is triethylamine,
the other symbols are as hereinbefore defined.

And the starting materials and reagents may be known per se or may be prepared by known methods.

The desired compound having hydroxy or amino may be easily prepared by a corresponding method selected from deprotection reactions such as deprotection under alkaline conditions, deprotection under acidic conditions and hydrogenolysis, using the compound having protected hydroxy or protected amino by a corresponding protecting group.

Methoxymethyl, tetrahydropyranyl, t-butyldimethylsilyl, acetyl, benzyl may be used as protecting groups for hydroxy. As protecting groups, other groups, which can be removed easily and selectively other than the above protecting groups, are also preferred.

Benzyloxycarbonyl, t-butoxycarbonyl, trifluoroacetyl may be used as protecting groups for amino. As protecting groups, other groups, which can be removed easily and selectively other than the above protecting groups, are also preferred. For example, the groups described in T.W. Greene, Protective Groups in Organic Synthesis, Wiley, New York, 1991, may be used.

In each reaction in the present specification, reaction products may be purified by conventional purification techniques, e.g. by distillation under atmospheric or reduced pressure, by high performance liquid chromatography, by thin layer chromatography or by column chromatography using silica gel or magnesium silicate; or by washing or by recrystallization. Purification may be carried out after each reaction or after a series of reactions.

### [Pharmacological Activities]

The compounds of the present invention of formula (I) or formula (I-A) bind strongly and show an antagonizing activity on the subtype(s) of PGE₂ receptor, EP₃ and /or EP₄ receptor.

For example, in a standard laboratory test, such effects of the compound of the present invention were confirmed by binding assay using the cell expressing the prostanoid receptor subtypes.
(i) Binding assay using cell expressing the prostanoid receptor subtypes
The preparation of membrane fraction was carried out according to the method of Sugimoto et al [J. Biol. Chem. 267, 6463-6466 (1992)], using CHO cell expressing prostanoid receptor subtypes (mouse EP₁, EP₂, EP_{3α}, and EP₄).
The standard assay mixture containing membrane fraction (50 µL), [³H]-PGE₂ in a final volume of 150 µL was incubated for 1 hour at room temperature. The reaction was terminated by addition of 3 mL of ice-cold buffer. The mixture was rapidly filtered through a glass filter (GF/B) under reduced pressure. The radioactivities associated with the filters were measured by liquid scintillation counter.
Kd and Bmax values were determined from Scatchard plots [Ann. N. Y. Acad. Sci. 51, 660(1949)]. Non-specific binding was determined as the amount bound in the presence of an excess (2.5 µM) of unlabeled PGE₂. In the experiment for competition of specific [³H]-PGE₂ binding assay, [³H]-PGE₂ was added at a concentration of 2.5 nM and a test compound of the present invention was added at various concentrations. The following buffer was used in all reactions.
Buffer: 10 mM potassium phosphate (pH 6.0), 1 mM EDTA, 10 mM MgCl₂, 0.1 M NaCl.
The inhibition constant (Ki) (µM) of each compound was calculated by the following equation. The results are shown in Table 1.

Ki=IC₅₀/(1+([C]/Kd))

**Table 1**

| | Ki(µM) | | | |
|---|---|---|---|---|
| Example No. | EP₁ receptor | EP₂ receptor | EP₃ receptor | EP₄ receptor |
| 2(1) | 1.5 | 8.0 | 0.06 | 0.01 |

(ii) EP₃ antagonizing activity assay using the cell expressing the prostanoid receptor subtypes
The preparation of CHO cell expressing mouse EP₃ receptor subtype was carried out according to the method of Sugimoto et al [J. Biol. Chem. 267, 6463-6466 (1992)]. The cells were cultured in 96-well microplates (10⁴ cells/well) for two days before experiments. After washing each well with 100 µL of PBS, Fura-2AM was added to taken in the cell for 60 minutes. After washing each well with HEPES, then a test compound and PGE₂ (10nM) were added at 37°C. A variation of intracellular calcium concentration was measured. Namely, excitation with a wavelength of 340/380 nm carried out, and fluorescence of 510 nm was measured, then a ratio of fluorescence intensity was calculated. By the way, an antagonizing activity of a test compound was calculated as inhibitory rate on the condition using PGE₂ (10 nM) as an agonist, and then IC₅₀ value was calculated.
(iii) EP₄ antagonizing activity assay using the cell expressing the prostanoid receptor subtypes
The preparation of CHO cell expressing mouse EP₄ receptor subtype was carried out according to the method of Nishigaki et al [FEBS lett., 364, 339-341(1995)]. The cells were cultured in 24-well microplates (10⁵ cells/well) for two days before experiments. After washing each well with 500 µL of MEM (minimum essential medium), thereto was added 450 µL of assay medium (MEM containings 1 mmol/L IBMX, 1%BSA), and the mixture was incubated for 10 minutes at 37 °C. Then PGE₂ alone or a combination with a test compound (50 µL) were added, and the mixture was incubated for 10 minutes at 37 °C. And reaction was terminated by addition of ice-cold TCA (10% w/v, 500 µL). This reaction mixture was freezed once (-80 °C) and thawed, and cells were harvested using a scraper. After centrifugation (13,000 r.p.m., for 3 minutes), cAMP content was measured using cAMP assay kit. That is, the supernatant (125 µL) was diluted with 500 µL of [¹²⁵I]-cAMP assay kit buffer (Amersham), and mixed with 0.5 mol/L tri-n-octylamine / chloroform solution (1 mL) was mixed. After removal of TCA from chloroform layer, cAMP content in the aqueous layer was quantified according to the method of kit manuals.
An antagonizing activity of compound (IC₅₀ value) was calculated as an inhibitory rate on the condition using 100nM PGE₂ as an agonist. This concentration of PGE₂ served a submaximal effect on cAMP production.
As mentioned above, it was clear that the compounds of the present invention show a strong antagonizing activity on the EP₃ and / or EP₄ subtype receptor.

### [Toxicity]

The toxicity of the compounds of the formula (I) or formula (I-A) of the present invention is very low and therefore, it is confirmed that these compounds are safe for use as medicine.

### [Application to Pharmaceuticals]

The compounds of the present invention of the formula (I) or formula (I-A) can bind and show the antagonizing activity on the PGE₂ receptor. Particularly, they bind to EP₃ receptor and/or EP₄ receptor strongly and show the antagonizing activity, are useful for the prevention and/or treatment of diseases induced by excess activation of EP₃ receptor and/or EP₄ receptor, for example, pain such as pain such as cancerous pain, fractural pain, pain following surgical and dental procedures; allodynia, hyperalgesia, pruritus, urticaria, atopic dermatitis, contact dermatitis, allergic conjunctivitis, various symptoms by treating with dialysis, asthma, rhinitis, sneeze, urinary frequency, neurogenic bladder, urinary disturbance, ejaculatory failure, defervescence, systemic inflammatory response syndrome, learning disturbance, Alzheimer' s disease, cancer such as formulation of cancer, growth of cancer and metastasis of cancer; retinopathy, patch of red, scald, burn, burn by steroid, renal failure, nephropathy, acute nephritis, chronic nephritis, abnormal blood levels of electrolytes, threatened premature delivery, abortion threatened, hypermenorrhea, dysmenorrhea, uterine fibroids, premenstrual syndrome, reproductive disorder, stress, anxiety disorders, depression, psychosomatic disorder, mental disorder, thrombosis, embolism, transient ischemia attack, cerebral infarction, atheroma, organ transplant, myocardial infarction, cardiac failure, hypertension, arteriosclerosis, circulatory failure and circulatory failure induced ulcer, neuropathies, vascular dementia, edema, various arthritis, rheumatism, diarrhea, constipation, disorder of bilious excretion, ulcerative colitis, Crohn's disease and / or bone diseases such as osteoporosis, rheumatoid arthritis, osteoarthritis, abnormal bone formation; cancer such as formation of cancer, proliferation of cancer, metastasis of cancer to organs and to bones and hypercalcemia induced metastasis to bones of cancer; systemic granuloma, immunological diseases such as ALS, multiple sclerosis, Sjoegren's syndrome, systemic lupus erythematosus, AIDS; allergy such as conjunctivitis, rhinitis, contact dermatitis, psoriasis; atopic dermatitis, asthma, pyorrhea, gingivitis, periodontitis, neuronal cell death, Alzheimer' s disease's disease, pulmonary injury, hepatopathy, acute hepatopathy, nephritis, renal failure, myocardial ischemia, Kawasaki disease, scald, ulcerative colitis, Crohn's disease, multiple organ, sleeping disorder and platelet aggregation.

For the purpose described above, the compounds of formula (I), of the present invention, non-toxic salts thereof may be normally administered systemically or topically, usually by oral or parenteral administration.

The doses to be administered are determined depending upon, for example, age, body weight, symptom, the desired therapeutic effect, the route of administration, and the duration of the treatment, etc. In the human adult, the doses per person at a time are generally from 0.1 mg to 100 mg, by oral administration, up to several times per day, and from 0.01 mg to 10 mg, by parenteral administration (preferably intravenous administration), up to several times per day, or continuous administration between 1 and 24 hours per day into vein.

As mentioned above, the doses to be used depend upon various conditions. Therefore, there are cases wherein doses lower than or greater than the ranges specified above may be used.

The compounds of the present invention may be administered in the form of, for example, solid compositions, liquid compositions or other compositions for oral administration, injections, liniments or suppositories for parenteral administration.

Solid compositions for oral administration include compressed tablets, pills, capsules, dispersible powders and granules. Capsules include hard capsules and soft capsules.

In such solid forms, one or more of the active compound(s) may be admixed with vehicles (such as lactose, mannitol, glucose, microcrystalline cellulose, starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof.

Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms, for example, freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se. Sprays may comprise additional substances other than diluents, such as stabilizing agents (such as sodium sulfate), isotonic buffers (such as sodium chloride, sodium citrate or citric acid). For preparation of such sprays, for example, the method described in the United States Patent No. 2,868,691 or 3,095,355 may be used.

### Best Mode for carrying out the Invention

The following reference examples and examples illustrate the present invention, but do not limit the present invention.

The solvents in the parentheses show the eluting or developing solvents and the ratios of the solvents used are by volume in chromatographic separations or TLC.

The solvents in the parentheses in NMR show the solvents used in measurement.

### Reference Example 1

2-(2-Nitro-4-cyanobenzyl)benzoic acid methyl ester

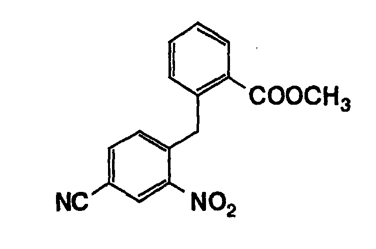

Under atmosphere of argon, to a solution of zinc powder (1.89 g) in anhydrous THF (15 ml) was added dibromoethane (116 *µ*l) and the mixture was stirred for five minutes at 60 °C. To the reaction mixture was added a solution of 2-bromomethylbenzoic acid methyl ester (4.42 g) in anhydrous THF (15 ml) over a period of 30 minutes at 0 °C and the mixture was stirred for 2 hours at the temperature to give a 2-carbomethoxybenzylzinc (II) bromide (benzyl zinc) solution.

Under atmosphere of argon, to a solution of 1-cyano-3-nitro-4-iodobenzene (2.00 g), bis(dibenzylideneacetone)palladium (42 mg) and 1,1'-bis(diphenylphosphino)ferrocene (41 mg) in anhydrous THF (10 ml) was added the above prepared benzyl zinc solution dropwise at room temperature and the mixture was stirred for 2 hours at 60 °C. To the reaction mixture was added a saturated aqueous solution of ammonium chloride at 0 °C and was extracted with ethyl acetate. The organic layer was washed with water and a saturated aqueous solution of sodium chloride and was dried over magnesium sulfate and was concentrated. The residue was purified by column chromatography on silica gel (n-hexane : ethyl acetate = 5 : 1) to give the title compound (809 mg) having the following physical data.
TLC: Rf 0.61 (n-hexane : ethyl acetate = 2 : 1);
NMR (300MHz, CDCl₃) : δ 8.25 (d, J = 1.8 Hz, 1H), 8.05 (dd, J = 7.8, 1.5 Hz, 1H), 7.66 (dd, J = 8.0, 1.8 Hz, 1H), 7.54 (dt, J = 7.8, 1.5 Hz, 1H), 7.42 (dt, J = 7.8, 1.5 Hz, 1H), 7.23 (d, J = 7.8 Hz, 1H), 7.10 (d, J = 8.0 Hz, 1H), 4.69 (s, 2H), 3.76 (s, 3H).

### reference Example 2

2-(2-Amino-4-cyanobenzyl)benzoic acid methyl ester

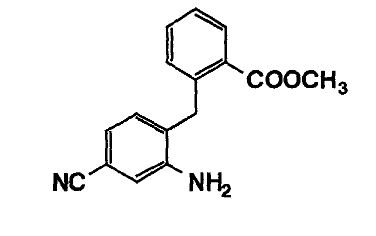

To a solution of the compound prepared in reference example 1 (705 mg) in acetic acid / water (7 ml / 0.7 ml) was added iron (664 mg) and the mixture was stirred for 20 minutes at 60 °C. To the reaction mixture was added water at 0 °C and was filtered over celite (brand name). The filtrate was washed with water, a saturated aqueous solution of sodium bicarbonate and a saturated aqueous solution of sodium chloride, dried over magnesium sulfate and was concentrated. The residue was purified by column chromatography on silica gel (hexane : ethyl acetate = 6 : 1 ∼ 4 : 1) to give the title compound (355 mg) having the following physical data.
TLC: Rf 0.40 (n-hexane : ethyl acetate = 2 : 1);
NMR (300MHz, CDCl₃) : δ 7.93 (dd, J = 7.7, 1.4 Hz, 1H), 7.44 (dt, J = 7.7, 1.4 Hz, 1H), 7.32 (dt, J = 7.7, 1.4 Hz, 1H), 7.14 (d, J = 7.8 Hz, 1H), 7.02-6.94 (m, 2H), 6.89 (s, 1H), 4.25 (s, 2H), 4.13 (bs, 2H), 3.88 (s, 3H).

### Example 1

2-[4-Cyano-2-[4-methyl-2-(1-naphthyl)pentanoylamino]phenyl]methylbenzoic acid methyl ester

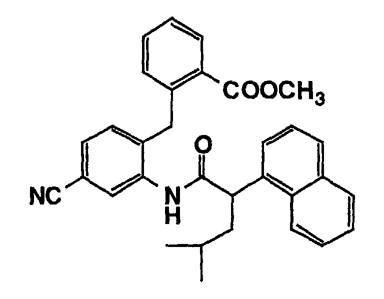

Under atmosphere of argon, the compound prepared in reference example 2 (213 mg) and pyridine (129 µl) in anhydrous methylene chloride (2 ml) was added 4-methyl-2-(1-naphthyl)pentanoyl chloride (250 mg) in anhydrous methylene chloride (1 ml) at 0 °C and the mixture was stirred for 15 minutes at the temperature. To the reaction mixture was added a saturated aqueous solution of sodium bicarbonate at 0 °C and was extracted with ethyl acetate. The organic layer was washed with water and a saturated aqueous solution of sodium chloride and was dried over sodium sulfate and was concentrated. The residue was purified by column chromatography on silica gel (n-hexane : ethyl acetate = 9 : 1 ∼ 3 : 1) to give the title compound (371 mg) having the following physical data.
TLC: Rf 0.60 (n-hexane : ethyl acetate = 2 : 1);
NMR (300MHz, CDCl₃) : δ 8.56 (bs, 1H), 8.07 (d, J = 7.8 Hz, 1H), 7.85 (m, 1H), 7.69 (d, J = 7.8 Hz, 1H), 7.58-7.45 (m, 2H), 7.37-7.21 (m, 6H), 7.18-7.05 (m, 2H), 6.87 (d, J = 7.8 Hz, 1H) , 4.42 (dd, J = 8.7, 5.4 Hz, 1H). 4.10 (d, J = 16 Hz, 1H), 3.87 (d, J = 16 Hz, 1H), 3.73 (s, 3H), 2.17 (m, 1H), 1.75-1.59 (m, 2H), 1.01 (d, J = 6.3 Hz, 3H), 0.92 (d, J = 6.3 Hz, 3H).

### Example 2

2-[4-Cyano-2-[4-methyl-2-(1-naphthyl)pentanoylamino]phenyl]methylbenzoic acid

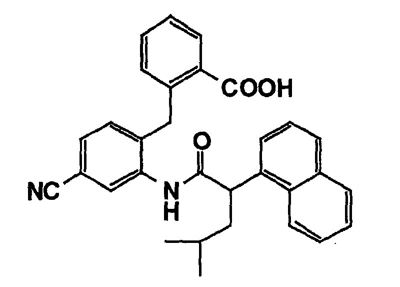

To a solution of the compound prepared in example 1 (365 mg) in THF / methanol (2 ml / 1 ml) was added 1N aqueous solution of sodium hydroxide (1 ml) and the mixture was stirred for 4 hours at room temperature. The reaction mixture was concentrated and the residue was diluted with ether and was extracted with water. The aqueous layer was neutralized with 1N hydrochloric acid and was extracted with ethyl acetate. The organic layer was washed with water and a saturated aqueous solution of sodium chloride, dried over magnesium sulfate and was concentrated. The residue was purified by column chromatography on silica gel (chloroform chloroform : methanol = 50 : 1) to give the title compound (313 mg) having the following physical data.
TLC: Rf 0.60 (chloroform ∼ methanol = 9 : 1);
NMR (300MHz, CDCl₃): δ 8.51 (s, 1H), 8.03 (d, J = 7.8 Hz, 1H), 7.95 (s, 1H), 7.82 (m, 1H), 7.68 (d, J = 7.8 Hz, 1H), 7.61 (d, J = 7.5 Hz, 1H), 7.53-7.40 (m, 2H), 7.36-7.06 (m, 6H), 6.79 (d, J = 7.5 Hz, 1H), 4.37 (t, J = 7.4 Hz, 1H), 4.09 (d, J = 16 Hz, 1H), 3.83 (d, J = 16 Hz, 1H), 2.18 (m, 1H), 1.80-1.54 (m, 2H), 0.98 (d, J = 6.6 Hz, 3H), 0.91 (d, J = 6.6 Hz, 3H).

### Example 2(1) ~ Example 2(53)

By the same procedure as described in Reference Example 1->Reference Example 2 -> Example 1 -> Example 2 using the corresponding compounds, optionally followed by converting to known salts, the following compounds were given.

### Example 2(1)

2-[2-[2-(4-Benzyloxyphenyl)propanoylamino]phenyl]methylbenzoic acid 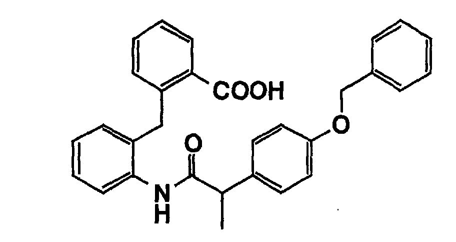 TLC: Rf 0.25 (n-hexane : ethyl acetate = 1 : 2);
NMR (300 MHz, CDCl₃: δ 8.01-7.97 (m, 2H), 7.47 (brs, 1H), 7.43-7.23 (m, 8H), 7.07-7.04 (m, 4H), 6.93 (d, J = 7.8 Hz, 1H), 6.80 (d, J = 8.4 Hz, 2H), 5.00 (s, 2H), 4.15 (d, J = 17.3 Hz, 1H), 4.14 (d, J = 17.3 Hz, 1H), 3.58 (q, J = 7.2 Hz, 1H), 1.45 (d, J = 7.2 Hz, 3H).

### Example 2(2)

2- [2-[2-(3-Benzyloxyphenyl)propanoylamino]phenyl]methylbenzoic acid 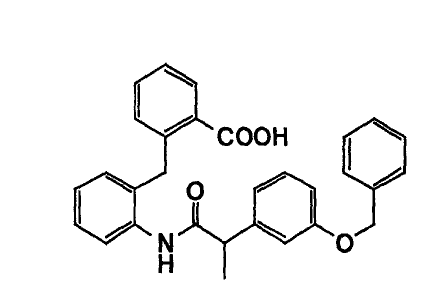 TLC: Rf 0.30 (n-hexane : ethyl acetate = 1 : 2);
NMR (300 MHz, CDCl₃): δ 7.96 (d, J = 8.1 Hz, 2H), 7.61 (brs, 1H), 7.40-7.22 (m, 8H), 7.11-7.04 (m, 3H), 6.98 (d, J = 7.5 Hz, 1H), 6.86 (s, 1H), 6.80-6.75 (m, 2H), 4.96 (s, 2H), 4.15 (d, J = 16.5 Hz, 1H), 4.13 (d, J = 16.5 Hz, 1H), 3.61 (q, J = 7.2 Hz, 1H), 1.47 (d, J = 7.2 Hz, 3H).

### Example 2(3)

2-[2-[2-(1-Naphthyl)butanoylamino]phenyl]methylbenzoic acid 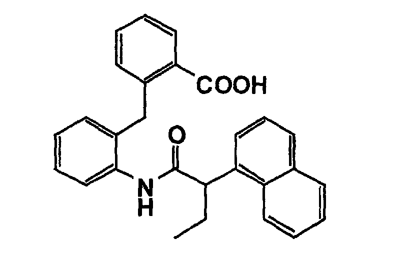 TLC: Rf 0.45 (n-hexane : ethyl acetate = 1 : 2);
NMR (300 MHz, CDCl₃) : δ 8.02 (d, J = 7.5 Hz, 2H), 7.80-7-72 (m, 2H), 7.66 (d, J = 8.1 Hz, 1H), 7.56 (brs, 1H), 7.45-7.40 (m, 2H), 7.32-7.14 (m, 5H), 7.07-7.00 (m, 2H), 6.80 (d, J = 7.5 Hz, 1H), 4.13 (t, J = 7.2 Hz, 1H), 4.05 (d, J = 16.8 Hz, 1H), 3.87 (d, J = 16.8 Hz, 1H), 2.34-2.25 (m, 1H), 1.98-1.89 (m, 1H), 0.95 (t, J = 7.2 Hz, 3H).

### Examble 2(4)

2-[2-[2-(1-Naphthyl)pentanoylamino]phenyl]methylbenzoic acid 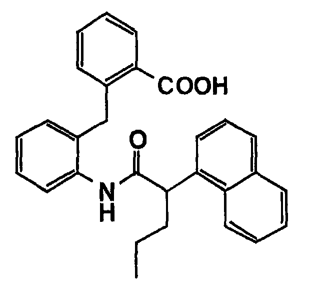 TLC: Rf 0.50 (n-hexane : ethyl acetate = 1 : 2);
NMR (300 MHz, CDCl₃) : δ 8.03 (d, J = 8.7 Hz, 2H), 7.81-7-77 (m, 1H), 7.73 (d, J = 6.6 Hz, 1H), 7.66 (d, J = 8.1 Hz, 1H), 7.57 (brs, 1H), 7.45-7.41 (m, 2H), 7.32-7.14 (m, 5H), 7.07-7.00 (m, 2H), 6.80 (d, J = 7.5 Hz, 1H), 4.23 (t, J = 7.4 Hz, 1H), 4.05 (d, J = 17.0 Hz, 1H), 3.87 (d, J = 17.0 Hz, 1H), 2.30-2.18 (m, 1H), 1.92-1.80 (m, 1H), 1.40-1.28 (m, 2H), 0.90 (t, J = 7.4 Hz, 3H).

### Example 2(5)

2-[2-[3-Methyl-2-(1-naphthyl)butanoylamino]phenyl]methylbenzoic acid 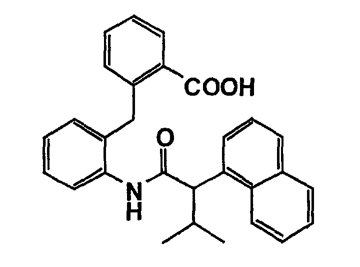 TLC: Rf 0.50 (n-hexane : ethyl acetate = 1 : 2);
NMR (300 MHz, CDCl₃) : δ 8.13 (d, J = 7.8 Hz, 1H), 7.93 (d, J = 8.1 Hz, 1H),7.81-7-66 (m, 4H), 7.55 (d, J = 7.5 Hz, 1H), 7.47-7.39 (m, 2H), 7.36-7.17 (m, 4H), 7.04-7.03 (m, 2H), 6.90 (d, J = 7.5 Hz, 1H), 4.15 (d, J = 16.5 Hz, 1H), 4.02 (d, J = 16.5 Hz, 1H), 3.91 (d, J = 9.9 Hz, 1H), 2.62-2.50 (m, 1H), 1.13 (d, J = 6.6 Hz, 3H), 0.70 (d, J = 6.6 Hz, 3H).

### Example 2(6)

2-[2-[2-(1,1'-Biphenyl-2-yl)propanoylamino]phenyl]methylbenzoic acid 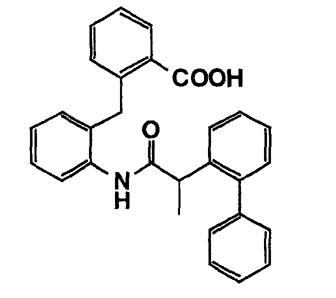 TLC: Rf 0.40 (n-hexane : ethyl acetate = 1 : 2);
NMR (300 MHz, CDCl₃) : δ 8.07 (dd, J = 7.5, 1.5 Hz, 1H), 7.90 (d, J = 8.1 Hz, 1H), 7.40-6.96 (m, 15H), 6.87 (d, J = 7.5 Hz, 1H), 4.07 (s, 2H), 3.78 (q, J = 6.9 Hz, 1H), 1.44 (d, J = 6.9 Hz, 3H).

### Example 2(7)

2-[2-[2-(1,1'-Biphenyl-3-yl)propanoylaminojphenyl]methylbenzoic acid 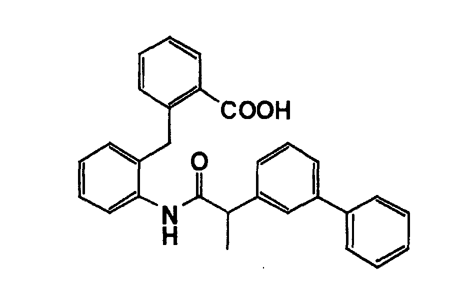 TLC: Rf 0.25 (n-hexane : ethyl acetate = 1 : 2);
NMR (300 MHz, CDCl₃) : δ 7.98 (d, J = 7.8 Hz, 1H), 7.89 (d, J = 7.8 Hz, 1H), 7.66 (br s. 1H), 7.51-7.04 (m, 14H), 6.97 (d, J = 7.5 Hz, 1H), 4.20 (d, J = 16.5 Hz, 1H), 4.10 (d, J = 16.5 Hz, 1H), 3.71 (q, J = 7.2 Hz, 1H), 1.53 (d, J = 7.2 Hz, 3H).

### Example 2(8)

2-[2-[2-(4-Phenoxyphenyl)propanoylamino]phenyl]methylbenzoic acid 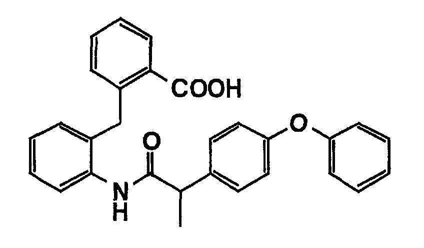 TLC: Rf 0.20 (n-hexane : ethyl acetate = 1 : 2);
NMR (300 MHz, CDCl₃) : δ 7.99 (d, J = 8.1 Hz, 1H), 7.94 (d, J = 7.8 Hz, 1H), 7.70 (br s. 1H), 7.42 (t, J = 7.5 Hz, 1H), 7.32-6.91 (m, 13H), 6.80 (d, J = 6.9 Hz, 1H), 4.17 (s, 2H), 3.61 (q, J = 7.2 Hz, 1H), 1.46 (d, J = 7.2 Hz, 3H).

### Example 2(9)

2-[2-[2-[4-(2-Phenylethoxy)phenyl]propanoylamino]phenyl]methylbenzoic acid 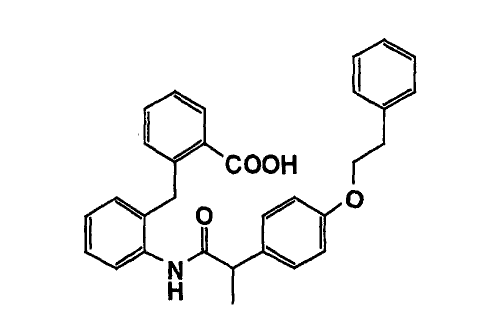 TLC: Rf 0.40 (n-hexane : ethyl acetate = 1 : 3);
NMR (300 MHz, CDCl₃) : δ 7.99 (d, J = 8.1 Hz, 1H), 7.95 (d, J = 8.1 Hz, 1H), 7.42 (brs, 1H), 7.27-7.19 (m, 9H), 7.06-7.01 (m, 3H), 6.89 (d, J = 7.2 Hz, 1H), 6.70 (d, J = 8.4 Hz, 2H), 4.20-4.07 (m, 4H), 3.56 (q, J = 7.2 Hz, 1H),
3.06 (t, J = 6.9 Hz, 2H), 1.44 (d, J = 7.2 Hz, 3H).

### Example 2(10)

2-[2-[2-[4-(3-Phenylpropoxy)phenyl]propanoylamino]phenyl]methylbenzoic acid 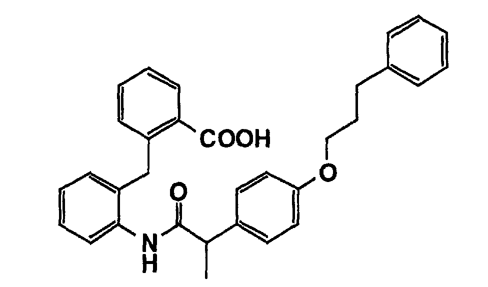 TLC: Rf 0.45 (n-hexane : ethyl acetate = 1:3);
NMR (300 MHz, CDCl₃): δ 7.98 (d, J = 8.1 Hz, 2H), 7.45 (brs, 1H), 7.37 (dt, J = 1.5, 7.5 Hz, 1H), 7.31-7.18 (m, 8H), 7.06-7.03 (m, 3H), 6.92 (d, J = 8.1 Hz, 1H), 6.70 (d, J = 8.4 Hz, 2H), 4.16 (d, J = 17. lHz, 1H), 4.14 (d, J = 17.1 Hz, 1H), 3.88 (t, J = 6.3 Hz, 2H), 3.57 (q, J = 7.2 Hz, 1H), 2.70 (t, J = 7.8 Hz, 2H), 2.12-2.02 (m, 2H), 1.44 (d, J = 7.2 Hz, 3H).

### Example 2(11)

2-[2-[2-Methoxy-2-(1-naphthyl)acetylamino]phenyl]methylbenzoic acid 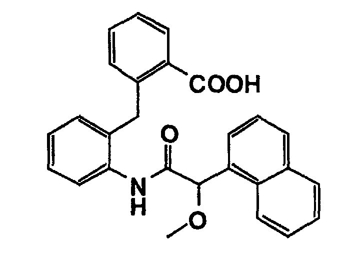 TLC: Rf 0.30 (n-hexane : ethyl acetate = 1 : 3);
NMR (300 MHz, CDCl₃) : δ 8.66 (brs, 1H), 8.17-8.12 (m, 2H), 8.04 (d, J = 8.1 Hz, 1H), 7.82-7-75 (m, 2H), 7.52-7.31 (m, 6H), 7.25-7.21 (m, 1H), 7.10-7.08 (m, 3H), 5.25 (s, 1H), 4.51 (s, 2H), 3.24 (s, 3H).

### Example 2(12)

2-[2-[2-(4-Isobutylphenyl)propanoylamino]phenyl]methylbenzoic acid 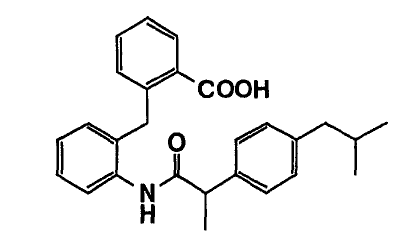 TLC: Rf 0.55 (ethyl acetate);
NMR (300 MHz, CDCl₃) : δ 8.02 (dd, J = 7.8, 1.2 Hz, 1H), 7.96 (d, J = 7.5 Hz, 1H), 7.50 (brs, 1H), 7.40 (dt, J = 1.5, 7.5 Hz, 1H), 7.33-7.21 (m, 2H), 7.10-6.98 (m, 7H), 4.12 (s, 2H), 3.62 (q, J = 6.9 Hz, 1H), 2.39 (d, J = 6.9 Hz, 2H), 1.81-1.72 (m, 1H), 1.48 (d, J = 6.9 Hz, 3H), 0.82 (d, J = 6.6 Hz, 6H).

### Example 2(13)

2-[2-[2-[4-(2-Thienyl)carbonylphenyl]propanoylamino]phenyl]methylbenzoic acid 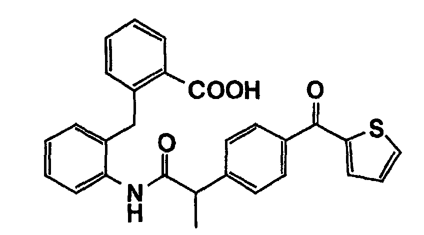 TLC: Rf 0.25 (ethyl acetate);
NMR (300 MHz, CDCl₃): δ 7.97-7.93 (m, 2H), 7.78 (brs, 1H), 7.75-7.72 (m, 3H), 7.62 (dd, J = 3.9, 0.9 Hz, 1H), 7.41-7.33 (m, 3H), 7.28-7.22 (m, 2H), 7.14 (dd, J = 4.8, 3.9 Hz, 1H), 7.09-7.03 (m, 3H), 4.15 (d, J = 16.5 Hz, 1H), 4.09 (d, J = 16.5 Hz, 1H), 3.75 (q, J = 7.2 Hz, 1H), 1.53 (d, J = 7.2 Hz, 3H).

### Example 2(14)

2-[2-[4-Methyl-2-(1-naphthyl)pentanoylamino]phenyl]methylbenzoic acid 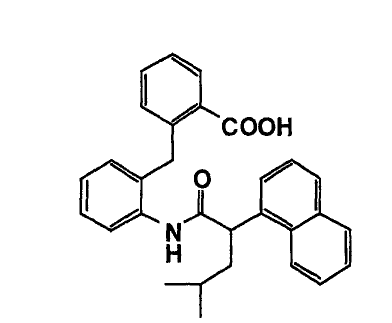 TLC: Rf 0.35 (n-hexane : ethyl acetate = 1 : 1);
NMR (300 MHz, CDCl₃) : δ 8.08-8.03 (m, 2H), 7.82-7.62 (m, 4H), 7.49-7.41 (m, 2H), 7.33-7.18 (m, 4H), 7.17-6.95 (m, 3H), 6.81 (d, J = 7.5 Hz, 1H), 4.34 (brt, 1H), 4.08 (d, J = 16.5 Hz, 1H), 3.84 (d, J = 16.5 Hz, 1H), 2.20- 2.12 (m, 1H), 1.77-1.69 (m, 1H), 1.64-1.56 (m, 1H), 0.95 (d, J = 6.5 Hz, 3H), 0.88 (d, J = 6.5 Hz, 3H).

### Example 2(15)

2-[2-[2-(1-Naphthyl)hexanoylamino]phenyl]methylbenzoic acid 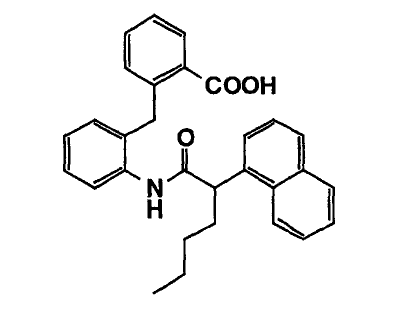 TLC: Rf 0.34 (n-hexane : ethyl acetate = 1 : 1);
NMR (300 MHz, CDCl₃) : δ 8.04 (d, J = 8.0 Hz, 2H), 7.80 (d, J = 7.0 Hz, 1H), 7.72-7.60 (m, 3H), 7.49-7.39 (m, 2H), 7.32-7.14 (m, 5H), 7.07-7.00 (m, 2H), 6.81 (d, J = 7.5 Hz, 1H), 4.21 (t, J = 7.5 Hz, 1H), 4.13 (d, J = 16. 5 Hz, 1H), 3.86 (d, J = 16.5 Hz, 1H), 2.33-2.19 (m, 1H), 1.94-1.79 (m, 1H), 1.40-1.20 (m, 4H), 0.84 (t, J = 7.0 Hz, 3H).

### Example 2(16)

2-[2-[3-Cyclopropyl-2-(1-naphthyl)propanoylamino]phenyl]methylbenzoic acid 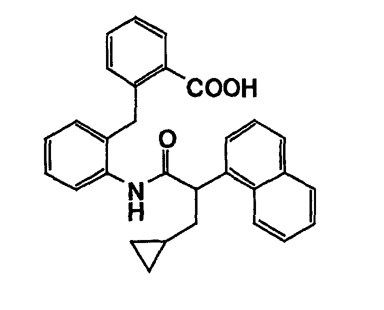 TLC: Rf 0.32 (n-hexane : ethyl acetate = 1 : 1);
NMR (300 MHz, CDCl₃): δ 8.08-8.04 (m, 2H), 7.81 (d, J = 7.0 Hz, 1H), 7.68-7.63 (m, 3H), 7.49-7.41 (m, 2H), 7.34 (d, J = 6.5 Hz, 1H), 7.30-7.18 (m, 3H), 7.14 (t, J = 7.0 Hz, 1H), 7.08-7.02 (m, 2H), 6.83 (d, J = 7.0 Hz, 1H ), 4.36 (t, J = 7.0 Hz, 1H), 4.10 (d, J = 16.5 Hz, 1H), 3.85 (d, J = 16.5 Hz, 1H), 2.16-2.06 (m, 1H), 1.88-1.79 (m, 1H), 0.74-0.65 (m, 1H), 0.42-0.32 (m, 2H), 0.16-0.02 (m, 2H).

### Example 2(17)

2-[2-[2-Cyclopropyl-2-(1-naphthyl)acetylamino]phenyl]methylbenzoic acid 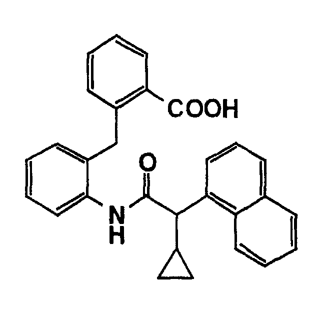 TLC: Rf 0.25 (n-hexane : ethyl acetate = 1: 1);
NMR (300 MHz, CDCl₃): δ 8.00-7.95 (m, 3H), 7.79-7.76 (m, 1H), 7.69 (d, J = 8.0 Hz, 1H), 7.53 (d, J = 7.0 Hz, 1H), 7.47-7.38 (m, 2H), 7.37-7.19 (m, 5H), 7.03 (t, J = 7.5 Hz, 1H), 6.96 (d, J = 7.0 Hz, 1H), 6.81 (d, J = 7.5 Hz, 1H), 3.95 (s, 2H), 3.53 (d, J = 9.0 Hz, 1H), 1.49-1.39 (m, 1H), 0.80-0.70 (m, 1H), 0.67-0.54 (m, 2H), 0.23-0.12 (m, 1H).

### Example 2(18)

**2**- [2-[2-[4-(4-Phenylbutoxy)phenyl]propanoylamino]phenyl]methylbenzoic acid 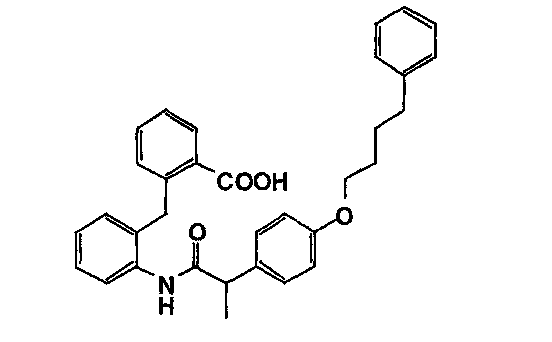 TLC: Rf 0.60 (ethyl acetate);
NMR (300 MHz, CDCl₃): δ 7.99 (t, J = 7.2 Hz, 2H), 7.45 (s, 1H), 7.37 (dt, J = 1.5, 7.5 Hz, 1H), 7.30-7.15 (m, 8H), 7.06-7.03 (m, 3H), 6.92 (d, J = 7.5 Hz, 1H), 6.69 (d, J = 8.4 Hz, 2H), 4.16 (d, J = 17.1Hz, 1H), 4.15 (d, J = 17.1 Hz, 1H), 3.88 (m, 2H), 3.57 (q, J = 6.9 Hz, 1H), 2.66 (m, 2H), 1.77 (m, 4H), 1.44 (d, J = 6.9 Hz, 3H).

### Example 2(19)

2-[2-[2-(4-Benzoylphenyl)propanoylamino]phenyl]methylbenzoic acid 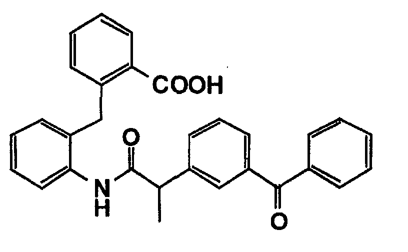 TLC: Rf 0.50 (ethyl acetate);
NMR (300 MHz, CDCl₃): δ 8.72 (s, 1H), 8.06 (s, 1H), 7.99 (dd, J = 8.1, 1.5 Hz, 1H), 7.92 (dd, J = 8.1, 1.5 Hz, 1H), 7.85 (d, J = 6.9 Hz, 2H), 7.63 (t, J = 7.5 Hz, 2H), 7.56-7.25 (m, 7H), 7.18-7.14 (m, 2H), 7.03-6.97 (m, 1H), 4.42 (d, J = 15.0 Hz, 1H), 4.04 (d, J = 15.0 Hz, 1H), 3.89 (q, J = 6.9 Hz, 1H), 1.60 (d, J = 6.9 Hz, 3H).

### Example 2(20)

2-[2-[2(R)-(1-Naphthyl)propanoylamino]phenyl]methylbenzoic acid 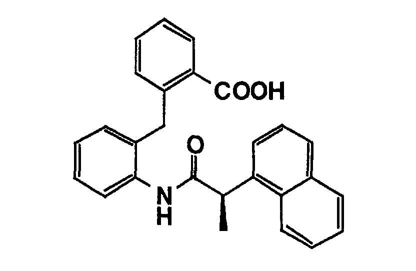 TLC: Rf 0.40 (ethyl acetate);
NMR (300 MHz, CDCl₃) : δ 8.00 (d, J = 8.1 Hz, 2H), 7.82-7.76 (m, 2H), 7.66 (d, J = 7.5 Hz, 1H), 7.45-7.39 (m, 3H), 7.31-7.16 (m, 5H), 7.06-6.95 (m, 2H), 6.77 (d, J = 7.2 Hz, 1H), 4.40 (q, J = 7.2 Hz, 1H), 3.94 (d, J = 16.8 Hz, 1H), 3.81 (d, J = 16.8 Hz, 1H), 1.66 (d, J = 7.2 Hz, 3H).

### Example 2(21)

2-[2-[2(S)-(1-Naphthyl)propanoylamino]phenyl]methylbenzoic acid 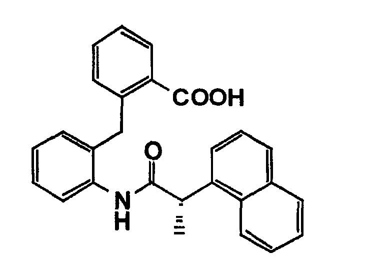 TLC: Rf 0.40 (ethyl acetate);
NMR (300 MHz, CDCl₃): δ 8.04-8.00 (m, 2H), 7.81-7.75 (m, 2H), 7.67 (d, J = 9.0 Hz, 1H), 7.47-7.42 (m, 3H), 7.28-7.16 (m, 5H), 7.06-6.97 (m, 2H), 6.79 (d, J = 7.8 Hz, 1H), 4.41 (q, J = 7.2 Hz, 1H), 3.96 (d, J = 16.8 Hz, 1H), 3.81 (d, J = 16.8 Hz, 1H), 1.65 (d, J = 7.2 Hz, 3H).

### Example 2(22)

2-[4-cyano-2-[4-methyl-2-(1-naphthyl)pentanoylamino]phenyl]methylbenzoic acid sodium salt 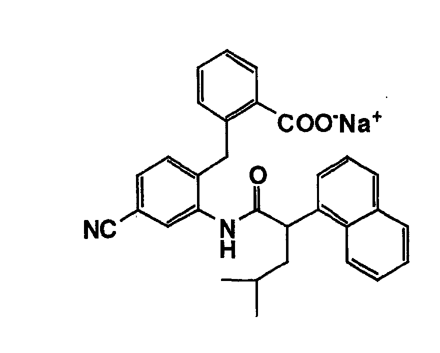 TLC: Rf 0.60 (chloroform : methanol = 9 : 1);
NMR (300 MHz, d₆-DMSO) : δ 8.63 (d, J = 8.4 Hz, 1H), 8.50 (s, 1H), 7.84 (m, 1H), 7.73 (d, J = 7.8 Hz, 1H), 7.65-7.36 (m, 8H), 6.98 (m, 2H), 6.87 (m, 1H), 5.16 (m, 1H), 4.61 (d, J = 14.4 Hz, 1H), 4.22 (d, J = 14.4 Hz, 1H), 1.60 (m, 2H), 1.31 (m, 1H), 0.84 (d, J = 6.3 Hz, 3H), 0.73 (d, J = 6.3 Hz, 3H).

### Example 2(23)

2-[2-[3-Phenyl-2-(1-naphthyl)propanoylamino]phenyl]methylbenzoic acid 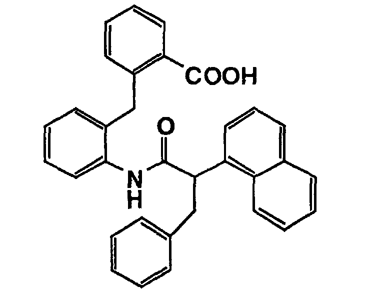 TLC: Rf 0.10 (n-hexane : ethyl acetate = 3 : 1);
NMR (200 MHz, CDCl₃): δ 8.10-7.96 (m, 2H), 7.79 (m, 1H), 7.73-7.60 (m, 2H), 7.34-6.98 (m, 11H), 6.83 (m, 1H), 4.49 (m, 1H), 4.06 (d, J = 16.4 Hz, 1H), 3.78 (d, J = 16.4 Hz, 1H), 3.67 (dd, J = 13.8, 8.0 Hz, 1H), 3.09 (dd, J = 13.8, 6.6 Hz, 1H).

### Example 2(24)

2-[2-[2-Ethoxy-2-(1-naphthyl)acetylamino]phenyl]methylbenzoic acid 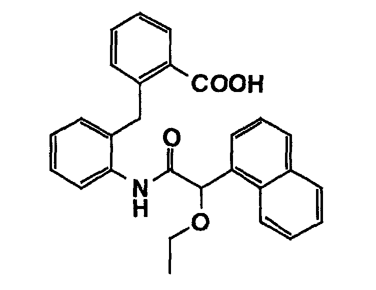 TLC: Rf 0.18 (n-hexane : ethyl acetate = 3 : 1);
NMR (300 MHz, CDCl₃): δ 8.77 (brs, 1H), 8.22-8.17 (m, 2H), 8.06 (d, J = 7.8 Hz, 1H),7.82-7.73 (m, 2H), 7.54-7.21(m, 6H), 7.13-7.03 (m, 3H), 5.40 (s, 1H), 4.53 (brs, 2H), 3.49-3.35 (m, 2H), 1.03(t, J = 7.2 Hz, 3H).

### Example 2(25)

2-[2-[2-Methoxymethoxy-2-(1-naphthyl)acetylamino]phenyl]methylbenzoic acid 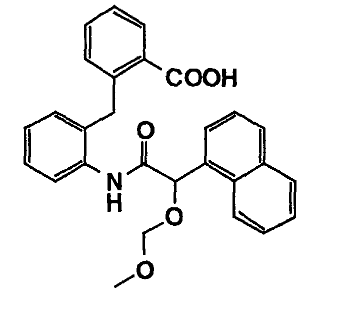 TLC: Rf 0.31 (n-hexane : ethyl acetate = 2 : 1);
NMR (300 MHz, CDCl₃): δ 8.87 (brs, 1H), 8.20-8.04 (m, 3H), 7.82-7.74 (m, 2H), 7.52-7.22 (m, 6H), 7.14-7.04 (m, 3H), 5.73 (s, 1H), 4.54-4.45 (m, 4H), 3.18 (s, 3H).

### Example 2(26)

2-[2-[2-(1-Naphthyl)heptanoylamino]phenyl]methylbenzoic acid 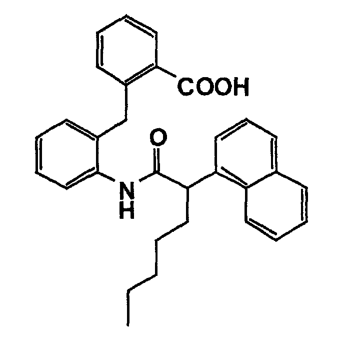 TLC: Rf 0.19 (n-hexane : ethyl acetate = 3 : 1);
NMR (300 MHz, CDCl₃): δ 8.09-7.98 (m, 2H), 7.80-7.72 (m, 2H), 7.65 (d, J = 6.9 Hz, 1H), 7.52 (bs, 1H), 7.49-7.37 (m, 2H), 7.35-7.13 (m, 4H), 7.08-6.97 (m, 2H), 6.79 (d, J = 6.9 Hz, 1H), 4.19 (m, 1H), 4.02 (brd, J = 17.1 Hz, 1H), 3.88 (brd, J = 17.1 Hz, 1H), 2.25 (m, 1H), 1.87 (m, 1H), 1.60-1.10 (m, 5H), 0.88-0.78 (m, 4H).

### Example 2(27)

2-[2-[3-Cyclobutyl-2-(1-naphthyl)propanoylamino]phenyl]methylbenzoic acid 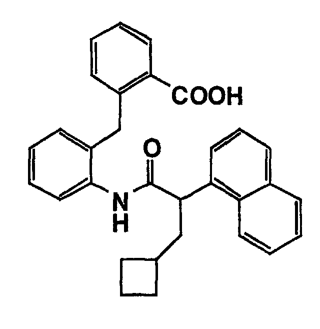 TLC: Rf 0.23 (n-hexane : ethyl acetate = 2 : 1);
NMR (200 MHz, CDCl₃) : δ 8.06-7.98 (m, 2H), 7.81-7.62 (m, 3H), 7.52-7.36 (m, 3H), 7.31-6.98 (m, 7H), 6.77 (brd, J = 7.6 Hz, 1H), 4.12 (m, 1H), 4.04 (d, J = 16.8 Hz, 1H), 3.84 (d, J = 16.8 Hz, 1H), 2.45-2.16 (m, 2H), 2.04-1.50 (m, 7H).

### Example 2(28)

2 - [2-[4,4-Dimethyl-2-(1-naphthyl)pentanoylamino]phenyl]methylbenzoic acid 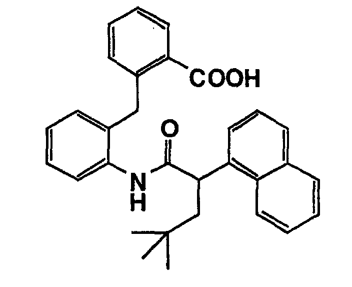 TLC: Rf 0.37 (n-hexane : ethyl acetate = 1 : 1);
NMR (300 MHz, CDCl₃): δ 8.10-8.00 (m, 2H), 7.85 (brs, 1H), 7.80 (m, 1H), 7.63 (brd, J = 7.8 Hz, 1H), 7.52-7.38 (m, 3H), 7.34 (brd, J = 7.6 Hz, 1H), 7.30-7.17 (m, 3H), 7.13-7.02 (m, 3H), 6.83 (brd, J = 7.8 Hz, 1H), 4.22 (m, 1H), 4.16 (d, J = 16.5 Hz, 1H), 3.85 (d, J = 16.5 Hz, 1H), 2.53 (m, 1H), 1.58 (dd, J = 13.8, 3.6 Hz, 1H), 0.92 (s, 9H).

### Example 2(29)

2-[2-[2-(4-Benzyloxymethylphenyl)propanoylamino]phenyl]methylbenzoic acid 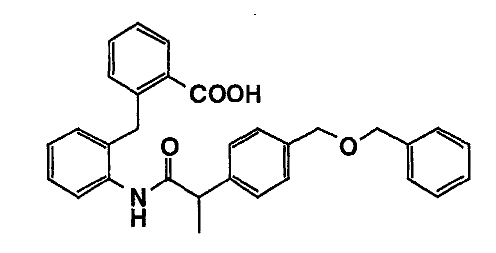 TLC: Rf 0.50 (chloroform : methanol = 10 : 1);
NMR (300 MHz, d₆-DMSO): δ 9.44 (s, 1H), 7.79 (d, J = 7.5 Hz, 1H), 7.42 (d, J = 7.8 Hz, 1H), 7.37-7.20 (m, 11H), 7.15 (t, J = 7.8 Hz, 1H), 7.04 (t, J = 7.8 Hz, 1H), 6.90 (d, J = 7.5 Hz, 2H), 4.48 (s, 2H), 4.46 (s, 2H), 4.24 (s, 2H), 3.84 (q, J= 6.9 Hz, 1H), 1.32 (d, J = 6.9 Hz, 3H).

### Example 2(30)

2-[2-[2-[4-(3-Phenylpropyl)phenyl]propanoylamino]phenyl]methylbenzoic acid 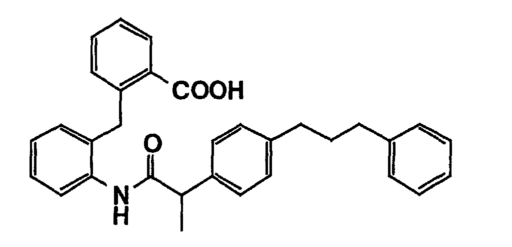 TLC: Rf 0.51 (chloroform : methanol = 10 : 1);
NMR (300 MHz, d₆-DMSO) : δ 9.41 (s, 1H), 7.79 (d, J = 7.2 Hz, 1H), 7.43 (d, J = 8.4 Hz, 1H), 7.36-7.00 (m, 13H), 6.89 (d, J = 7.5 Hz, 2H), 4.22 (s, 2H), 3.80 (q, J= 6.9 Hz, 1H), 2.60-2.40 (m, 4H), 1.82 (m, 2H), 1.30 (d, J = 6.9 Hz, 3H).

### Example 2(31)

2-[2-[2-(Quinolin-5-yl)propanoylamino]phenyl]methylbenzoic acid 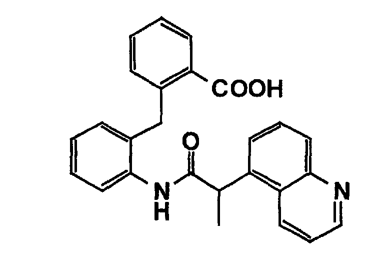 TLC: Rf 0.25 (chloroform : methanol = 9 : 1);
NMR (300 MHz, CD₃OD): δ 8.82 (dd, J = 4.2, 1.2 Hz, 1H), 8.63 (d, J = 8.7 Hz, 1H), 7.90 (d, J = 8.4 Hz, 1H), 7.66-7.50 (m, 5H),7.24-7.18 (m, 2H), 7.14-7.09 (m, 3H), 6.87 (d, J = 7.5 Hz, 1H), 4.60 (q, J = 7.2 Hz, 1H), 4.25 (d, J = 16.5 Hz, 1H), 4.10 (d, J = 16.5 Hz, 1H), 1.56 (d, J = 7.2 Hz, 3H).

### Example 2(32)

2-[2-[2-[4-[2-(3-Pyridyl)ethoxy]phenyl]propanoylamino]phenyl]methylbenzoic acid hydrochloride 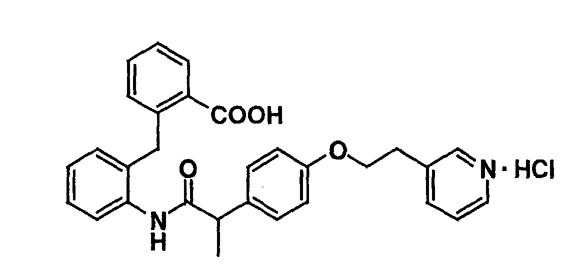 TLC: Rf 0.56 (chloroform : methanol = 9 : 1);
NMR (300 MHz, d₆-DMSO) : δ 9.39 (brs, 1H), 8.84 (brs, 1H), 8.75 (d, J = 5.4 Hz, 1H), 8.42 (d, J = 8.0 Hz, 1H), 7.91 (dd, J = 8.0, 5.4 Hz, 1H), 7.79 (dd, J = 8.0, 1.2 Hz, 1H), 7.42 - 7.10 (m, 6H), 7.04 (m, 1H), 6.92 - 6.86 (m, 2H), 6.81 (d, J = 8.7 Hz, 2H), 4.25 - 4.20 (m, 2H), 4.22 (s, 2H), 3.77 (q, J = 6.9 Hz, 1H), 3.20 (t, J = 6.2 Hz, 2H), 1.28 (d, J = 6.9 Hz, 3H).

### Example 2(33)

2-[2-[2-[4-(2-Phenoxyethyl)phenyl]propanoylamino]phenyl]methylbenzoic acid 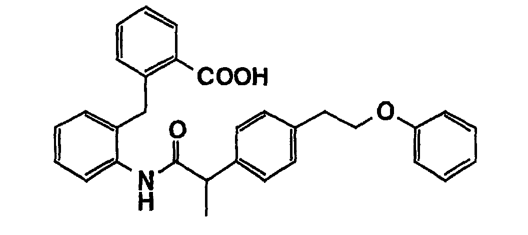 TLC: Rf 0.15 (n-hexane : ethyl acetate = 1:1);
NMR (300 MHz, CDCl₃): δ 7.99 (dd, J = 7.5, 1.2 Hz, 1H), 7.95 (brd, J = 8.1 Hz, 1H), 7.55 (brs, 1H), 7.36 (m, 1H), 7.30-7.21 (m, 4H), 7.15-7.02 (m, 6H), 6.98-9.83 (m, 4H), 4.21-4.08 (m, 4H), 3.61 (q, J = 7.2 Hz, 1H), 3.01 (t, J = 6.9 Hz, 2H), 1.45 (d, J = 7.2 Hz, 3H).

### Example 2(34)

2-[2-[4-Methoxy-2-(1-naphthyl)butanoylamino]phenyl]methylbenzoic acid 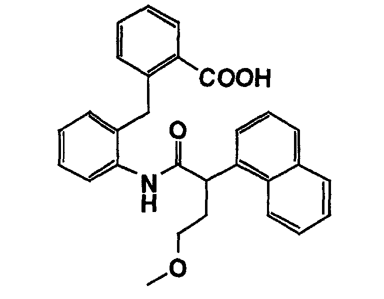 TLC: Rf 0.51 (chloroform : methanol = 10 : 1);
NMR (300 MHz, d₆-DMSO): δ 9.69 (s, 1H), 8.30 (d, J = 8.4 Hz, 1H), 7.91 (d, J = 7.5 Hz, 1H), 7.79 (d, J = 8.1 Hz, 1H), 7.74 (m, 1H), 7.59-7.19 (m, 6H), 7.15 (t, J = 7.5 Hz, 1H), 7.04 (t, J = 7.5 Hz, 1H), 6.88 (d, J = 8.1 Hz, 1H), 6.84 (d, J = 7.8 Hz, 1H), 4.69 (dd, J = 8.7, 5.1 Hz, 1H), 4.26 (d, J = 15.9 Hz, 1H), 4.18 (d, J = 15.9 Hz, 1H), 3.29 (s, 2H), 3.17 (s, 3H), 2.29 (m, 1H), 1.94 (m, 1H).

### Example 2(35)

2-[2-[5-Methyl-2-(1-naphthyl)hexanoylamino]phenyl]methylbenzoic acid 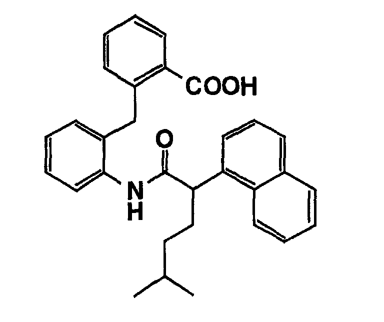 TLC: Rf 0.69 (chloroform : methanol = 10 : 1);
NMR (300 MHz, CDCl₃): δ 8.04 (d, J = 7.8 Hz, 2H), 7.80 (m, 1H), 7.74-7.64 (m, 2H), 7.61 (bs, 1H), 7.45 (m, 2H), 7.34-7.12 (m, 5H), 7.02 (m ,2H), 6.82 (d, J = 7.8 Hz, 1H) 4.17 (t, J = 7.2 Hz, 1H), 4.04 (d, J = 16.8 Hz, 1H), 3.87 (d, J = 16.8 Hz, 1H), 2.25 (m, 1H), 1.87 (m, 1H), 1.54 (m, 1H), 1.28 (m, 1H), 1.15 (m, 1H), 0.86 (d, J = 6.9 Hz, 3H), 0.84 (d, J = 6.9 Hz, 3H).

### Example 2(36)

2-[2-[2-[4-[N-Methyl-N-(2-phenylethyl)amino]phenyl]propanoylamino]phenyl]methylbenzoic acid hydrochloride 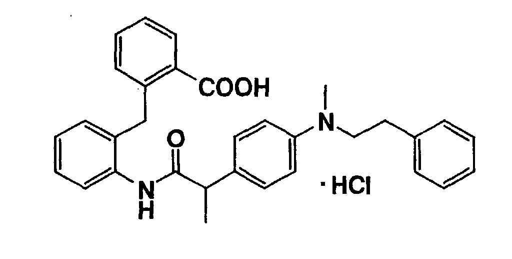 TLC: Rf 0.35 (chloroform : methanol = 9 : 1);
NMR (300 MHz, CD₃OD) : δ 7.80 (dd, 1H), 7.59-7-51 (m, 4H), 7.46 (dd, 1H), 7.34-7.09 (m, 9H), 7.04-6.97 (m, 2H), 4.20 (s, 2H), 3.92 (q, J = 7.2 Hz, 1H), 3.80 (t, J= 8.1 Hz, 2H), 3.26 (s, 3H), 2.74 (t, J = 8.1 Hz, 2H), 1.46 (d, J = 7.2 Hz, 3H).

### Example 2(37)

2-[2-[2-[4-[N-Acetyl-N-(2-phenylethyl)amino]phenyl]propanoylamino]phenyl]methylbenzoic acid 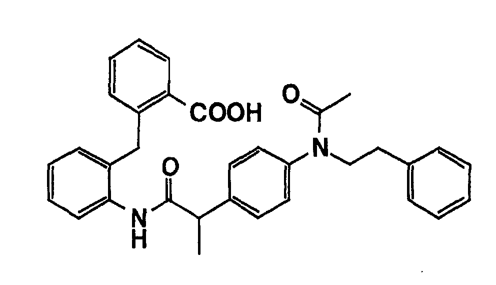 TLC: Rf 0.45 (chloroform : methanol = 9 : 1);
NMR (300 MHz, CDCl₃): δ 8.73 (s, 1H), 8.14 (d, J = 7.8 Hz, 1H), 8.00 (d, J = 7.5 Hz, 1H), 7.43-7.07 (m, 13H), 6.96 (d, J = 8.1 Hz, 2H), 4.48 (d, J = 15.6 Hz, 1H), 4.05 (d, J = 15.6 Hz, 1H), 3.91 (t, J = 7.8 Hz, 2H), 3.74 (q, J = 7.2 Hz, 1H), 2.86 (t, J = 7.8 Hz, 2H), 1.86 (s, 3H), 1.41 (d, J = 7.2 Hz, 3H).

### Example 2(38)

5-[2-[2-(1-Naphthyl)propanoylamino]phenyl]methyloxazole-4-carboxylic acid 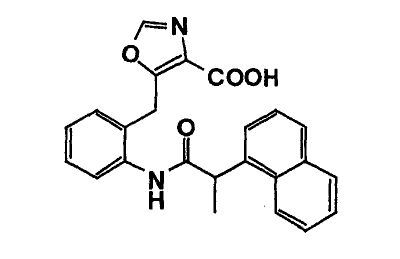 TLC: Rf 0.26 (chloroform : methanol = 9 : 1);.
NMR (300 MHz, d₆-DMSO) : δ 13.1 (br, 1H), 9.69 (s, 1H), 8.27 (d, J = 8.0 Hz, 1H), 8.24 (s, 1H), 7.93 (dd, J = 8.0, 1.8 Hz, 1H), 7.82 (d, J = 8.0 Hz, 1H), 7.60 - 7.45 (m, 4H), 7.29 (dd, J = 8.0, 1.2 Hz, 1H), 7.21 (m, 1H), 7,12 (m, 1H), 7.03 (dd, J = 8.0, 1.2 Hz, 1H), 4.65 (q, J = 7.2 Hz, 1H), 4.33 (d, J = 16.5 Hz, 1H), 4.26 (d, J = 16.5 Hz, 1H), 1.56 (d, J = 7.2 Hz, 3H).

### Example 2(39)

2-[2-[2-[4-[N-(2-Phenylethyl)amino]phenyl]propanoylamino]phenyl]methylbenzoic acid hydrochloride 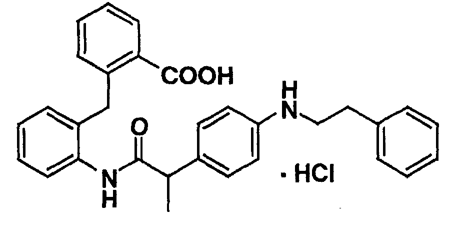 TLC: Rf 0.40 (chloroform : methanol = 9 : 1);
NMR (300 MHz, CD₃OD) : δ 7.80 (dd, J = 7.5, 1.2 Hz, 1H), 7.51-6.97 (m, 16H), 4.20 (s, 2H), 3.55 (q, J = 7.2 Hz, 1H), 3.55 (t, J = 8.1 Hz, 2H), 2.98 (t, J = 8.1 Hz, 2H), 1.44 (d, J = 7.2 Hz, 3H).

### Example 2(40)

2-[2-[2-[4-(2-Phenylethylthio)phenyl]propanoylamino]phenyl]methylbenzoic acid 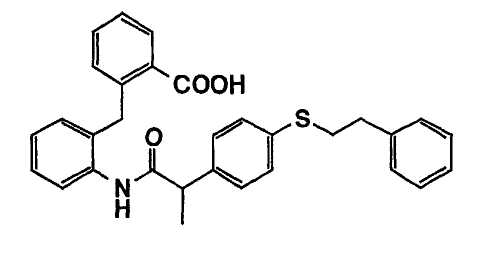 TLC: Rf 0.45 (chloroform : methanol = 9 : 1);
NMR (200 MHz, CDCl₃) : δ 7.98-7.95 (m, 2H), 7.63 (s, 1H), 7.42-6.96 (m, 15H), 4.18 (d, J = 16.9 Hz, 1H), 4.16 (d, J = 16.9 Hz, 1H), 3.60 (q, J = 7.2 Hz, 1H), 3.16-3.07 (m, 2H), 2.92-2.84 (m, 2H), 1.45 (d, J = 7.2 Hz, 3H).

### Example 2(41)

2-[2-[2-[4-(2-Phenylethylsulfinyl)phenyl]propanoylamino]phenyl]methylbenzoic acid 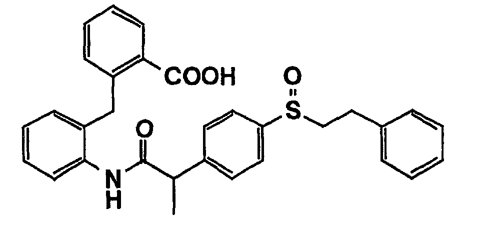 TLC: Rf 0.45 (chloroform : methanol = 9 : 1);
NMR (300 MHz, CDCl₃) : δ 8.59(brs, 0.5H), 8.38(brs, 0.5H), 8.07-8.02 (m, 1H), 7.93-7.91 (m, 1H), 7.48-6.93 (m, 15H), 4.25-4.12 (m, 2H), 3.78-3.70 (m, 1H), 3.09-2.88 (m, 4H), 1.38-1.33 (m, 3H).

### Example 2(42)

2-[2-[2-[4-(2-Phenylethylsulfonyl)phenyl]propanoylamino]phenyl]methylbenzoic acid 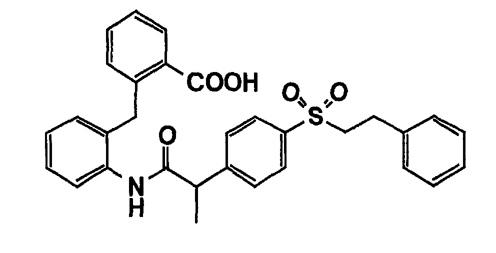 TLC: Rf 0.45 (chloroform : methanol = 9 : 1);
NMR (300 MHz, CDCl₃): δ 8.10(s, 1H), 7.99 (d, J = 8.1 Hz, 1H), 7.90 (d, J = 8.1 Hz, 1H), 7.89 (d, J = 8.4 Hz, 2H), 7.42-7.29 (m, 3H), 7.26-7.07 (m, 10H), 4.25 (d, J = 16.2 Hz, 1H), 4.14 (d, J = 16.2 Hz, 1H), 3.75 (q, J = 7.2 Hz, 1H), 3.36-3.31 (m, 2H), 3.06-3.00 (m, 2H), 1.48 (d, J = 7.2 Hz, 3H).

### Example 2(43)

2-[2-[2-(1-Naphthyl)-4-pentenoylamino]phenyl]methylbenzoic acid 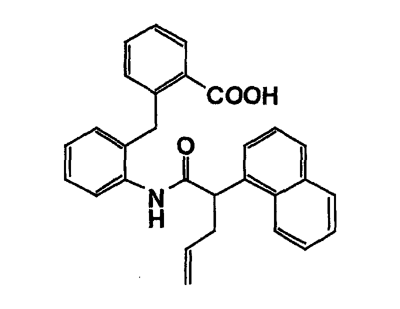 TLC: Rf 0.60 (chloroform : methanol = 10 : 1);
NMR (300 MHz, d₆-DMSO): δ 9.66 (s, 1H), 8.28 (d, J = 7.8 Hz, 1H), 7.92 (m, 1H), 7.80 (d, J = 8.7 Hz, 1H), 7.77 (m, 1H), 7.53-7.35 (m, 5H), 7.34-7.22 (m, 2H), 7.15 (m, 1H), 7.04 (m, 1H), 6.92-6.83 (m, 2H), 5.80 (m 1H), 5.10 (dd, J = 17.1, 1.8 Hz, 1H), 4.95 (dd, J = 10.2, 1.8 Hz, 1H), 4.64 (dd, J = 9.0, 6.0 Hz, 1H), 4.27 (d, J = 16.2 Hz, 1H), 4.20 (d, J = 16.2 Hz, 1H), 2.78 (m, 1H), 2.55 (m, 1H).

### Example 2(44)

2-[2-[2-(1-Naphthyl)-4-hexenoylamino]phenyl]methylbenzoic acid 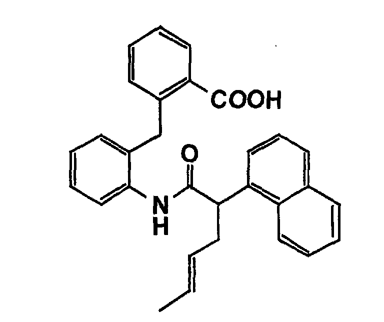 TLC: Rf 0.62 (chloroform : methanol = 10 : 1);
NMR (300 MHz, d₆-DMSO) : δ 9.67 (s, 1H), 8.29 (d, J = 8.1 Hz, 1H), 7.92 (m, 1H), 7.80 (d, J= 7.8 Hz, 1H), 7.77 (m, 1H), 7.64-7.21 (m, 7H), 7.15 (m, 1H), 7.03 (m, 1H), 6.93-6.81 (m, 2H), 5.50 (m 2H), 4.58 (dd, J = 8.7, 5.1 Hz, 1H), 4.28 (d, J = 16.8 Hz, 1H), 4.21 (d, J = 16.8 Hz, 1H), 2.75 (m, 1H), 2.53 (m, 1H), 1.52 (d, J = 5.4 Hz, 3H).

### Example 2(45)

2-[2-[4-Ethyl-2-(1-naphthyl)hexanoylamino]phenyl]methylbenzoic acid 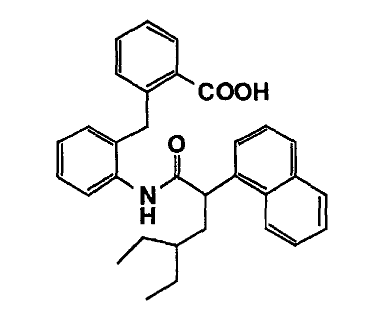 TLC: Rf 0.63 (chloroform : methanol = 10 : 1);
NMR (300 MHz, d₆-DMSO) : δ 9.63 (s, 1H), 8.34 (d, J = 8.1 Hz, 1H), 7.91 (d, J = 8.4 Hz, 1H), 7.78 (d, J = 9.3 Hz, 1H), 7.75 (m, 1H), 7.64 (d, J = 6.9 Hz, 1H), 7.60-7.40 (m, 3H), 7.34 (d, J = 7.8 Hz, 1H), 7.29-7.10 (m, 3H), 7.04 (m, 1H), 6.89-6.80 (m, 2H), 4.63 (dd, J = 9.0, 5.1 Hz, 1H), 4.29 (d, J = 16.5 Hz, 1H), 4.20 (d, J = 16.5 Hz, 1H), 1.99 (m, 1H), 1.61-1.12 (m, 6H), 0.77 (t, J = 7.2 Hz, 3H), 0.73 (t, J = 7.5 Hz, 3H).

### Example 2(46)

2-[2-[2-(1-Naphthyl)-4-pentynoylamino]phenyl]methylbenzoic acid 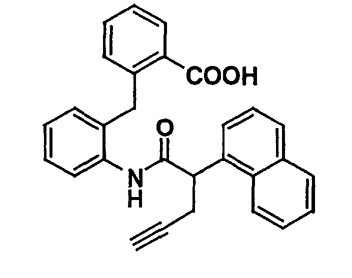 TLC: Rf 0.63 (chloroform : methanol = 10 : 1);
NMR (300 MHz, CDCl₃) : δ 8.08 (m, 1H), 7.97 (bs, 1H), 7.84 (m, 1H), 7.71 (d, J = 8.1 Hz, 1H), 7.55-7.44 (m, 3H), 7.37 (d, J = 7.2 Hz, 1H), 7.31-7.20 (m, 4H), 7.11 (d, J = 7.5 Hz, 1H), 7.06 (d, J = 4.2 Hz, 2H), 6.89 (d, J = 7.5 Hz, 1H), 4.53 (t, J = 7.5 Hz, 1H), 4.16 (d, J = 16.2 Hz, 1H), 3.81 (d, J = 16.2 Hz, 1H), 3.15 (ddd, J = 16.8, 7.5, 2.4 Hz, 1H), 2.76 (ddd, J = 16.8, 7.5, 2.4 Hz, 1H), 1.92 (t, J = 2.4 Hz, 1H).

### Example 2(47)

2-[2-[3-Methoxy-2-(1-naphthyl)propanoylamino]phenyl]methylbenzoic acid 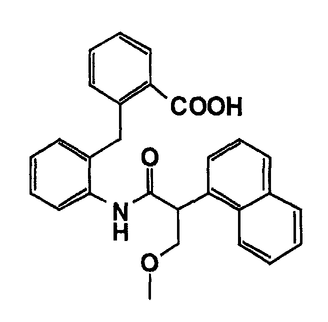 TLC: Rf 0.55 (chloroform : methanol = 10 : 1);
NMR (300 MHz, d₆-DMSO) : δ 9.77 (s, 1H), 8.34 (d, J = 8.4 Hz, 1H), 7.95 (d, J = 7.8 Hz, 1H), 7.84 (d, J = 8.1 Hz, 1H), 7.80-7.72 (m, 1H), 7.66-7.40 (m, 5H), 7.36-7.21 (m, 2H), 7.17 (t, J = 7.2 Hz, 1H), 7.06 (t, J = 7.2 Hz, 1H), 6.93 (d, J = 7.2 Hz, 1H), 6.88 (d, J = 7.2 Hz, 1H), 4.90 (dd, J = 9.0, 4.8 Hz, 1H), 4.27 (s, 2H), 4.00 (t, J = 9.0 Hz, 1H), 3.63 (dd, J = 9.0, 4.8 Hz, 1H), 3.29 (s, 3H).

### Example 2(48)

2-[4-Ethoxy-2-[4-methyl-2-(1-naphthyl)pentanoylamino]phenyl]methylbenzoic acid 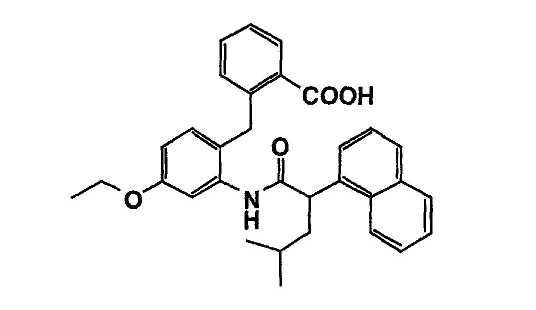 TLC: Rf 0.58 (chloroform : methanol = 10 : 1);
NMR (300 MHz, d₆-DMSO) : δ 9.59 (s, 1H), 8.34 (d, J = 8.4 Hz, 1H), 7.96 (d, J = 8.4 Hz, 1H), 7.83 (d, J = 7.8 Hz, 1H), 7.76 (d, J = 6.9 Hz, 1H), 7.69-7.43 (m, 4H), 7.33-7.21 (m, 2H), 7.12 (s, 1H), 6.90 (d, J = 7.8 Hz, 1H), 6.84 (d, J = 7.2 Hz, 1H), 6.70 (d, J = 8.4 Hz, 1H), 4.65 (m, 1H), 4.30 (d, J = 16.5 Hz, 1H), 4.17 (d, J = 16.5 Hz, 1H), 3.98 (q, J = 6.9 Hz, 1H), 1.95 (m, 1H), 1.62-1.40 (m, 2H), 1.32 (t, J = 6.9 Hz, 3H), 0.96 (d, J = 6.0 Hz, 3H), 0.85 (d, J = 6.0 Hz, 3H).

### Example 2(49)

2-[4-Isopropyloxy-2-[4-methyl-2-(1-naphthyl)pentanoylamino]phenyl]met hylbenzoic acid 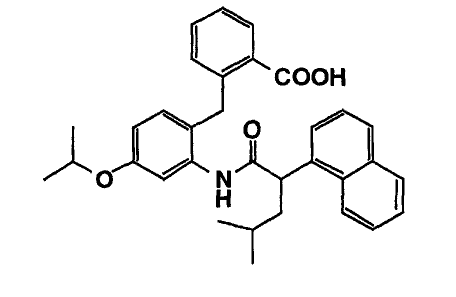 TLC: Rf 0.59 (chloroform : methanol = 10 : 1);
NMR (300 MHz, CDCl₃): δ 8.03 (d, J = 7.2 Hz, 1H), 7.86-7.75 (m, 2H), 7.70-7.59 (m, 3H), 7.50-7.39 (m, 2H), 7.30-7.08 (m, 4H), 6.92 (d, J = 8.4 Hz, 1H), 6.80 (d, J = 7.8 Hz, 1H), 6.58 (dd, J = 8.4, 2.4 Hz, 1H), 4.56 (m, 1H), 4.32 (t, J = 7.2 Hz, 1H), 4.01 (d, J = 16.8 Hz, 1H), 3.78 (d, J = 16.8 Hz, 1H), 2.14 (m, 1H), 1.73 (m, 1H), 1.59 (m, 1H), 1.32 (d, J = 6.3 Hz, 6H), 0.94 (d, J = 6.6 Hz, 3H), 0.88 (d, J = 6.6 Hz, 3H).

### Example 2(50)

5-[2-[4-Methyl-2-(1-naphthyl)pentanoylamino]phenyl]methyloxazole-4-carboxylic acid 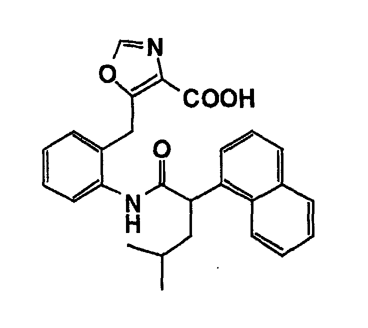 TLC: Rf 0.40 (chloroform : methanol = 9 : 1);
NMR (300 MHz, CDCl₃): δ 8.31 (d, J = 8.4 Hz, 1H), 8.08 (s, 1H), 7.89-7.65 (m, 4H), 7.60-7.40 (m, 4H), 7.28-7.18 (m, 2H), 7.05 (t, J = 7.2 Hz, 1H), 4.62 (t, J = 7.8 Hz, 1H), 4.03 (d, J = 16 Hz, 1H), 3.92 (d, J = 16 Hz, 111), 2.36 (m, 1H), 1.92 (m, 1H), 1.69 (m, 1H), 1.03 (d, J = 6.6 Hz, 3H), 0.98 (d, J = 6.6 Hz, 3H).

### Example 2(51)

2-[4-Cyano-2-[3-cyclopropyl-2-(1-naphthyl)propanoylamino]phenyl]methylbenzoic acid 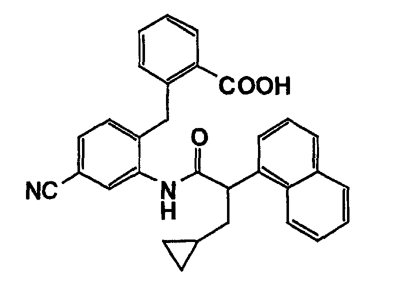 TLC: Rf 0.58 (chloroform : methanol = 10 : 1);
NMR (300 MHz, d₆-DMSO): δ 9.95 (s, 1H), 8.31 (m, 1H), 7.91 (m, 2H), 7.80 (d, J = 7.8 Hz, 2H), 7.65-7.21 (m, 7H), 7.00 (d, J = 7.8 Hz, 1H), 6.90 (d, J = 7.5 Hz, 1H), 4.71 (m, 1H), 4.41 (d, J = 16.8 Hz, 1H), 4.27 (d, J = 16.8 Hz, 1H), 1.97 (m, 1H), 1.63 (m, 1H), 0.68 (m, 1H), 0.34 (m, 2H), 0.10 (m, 2H).

### Example 2(52)

2-[2-[5-Methyl-2-(1-naphthyl)-4-hexenoylamino]phenyl]methylbenzoic acid 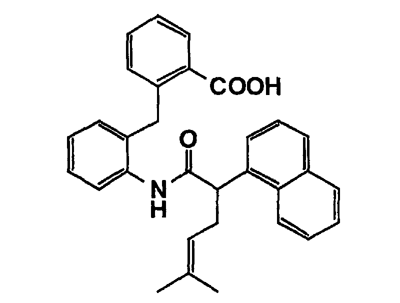 TLC: Rf 0.61 (chloroform : methanol = 10 : 1);
NMR (300 MHz, d₆-DMSO) : δ 9.67 (s, 1H), 8.30 (d, J = 8.4 Hz, 1H), 7.92 (m, 1H), 7.78 (m, 2H), 7.65-7.41 (m, 4H), 7.37-7.21 (m, 3H), 7.14 (m, 1H), 7.03 (m, 1H), 6.91 (d, J = 7.2 Hz, 1H), 6.83 (d, J = 7.2 Hz, 1H), 5.18 (m, 1H), 4.55 (m, 1H), 4.24 (s, 2H), 2.78 (m, 1H), 2.45 (m, 1H), 1.55 (s, 6H).

### Example 2(53)

2-[4-Methoxy-2-[4-methyl-2-(1-naphthyl)pentanoylamino]phenyl]methylb enzoic acid 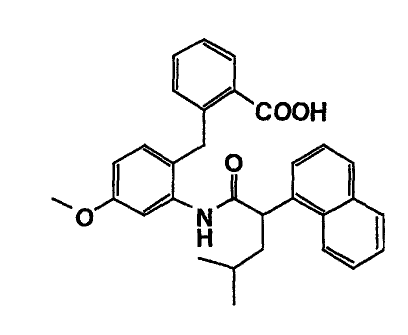 TLC: Rf 0.48 (chloroform : methanol = 10 : 1);
NMR (300 MHz, CDCl₃) :δ 8.04 (d, J = 7.8 Hz, 1H), 7.87 (d, J = 2.7 Hz, 1H), 7.83-7.72 (m, 2H), 7.69-7.56 (m, 2H), 7.46 (m, 2H), 7.30-7.17 (m, 3H), 7.11 (m, 1H), 6.94 (d, J = 8.7 Hz, 1H), 6.80 (d, J = 7.8 Hz, 1H), 6.60 (dd, J = 8.4, 2.4 Hz, 1H), 4.34 (t, J = 7.2 Hz, 1H), 4.03 (d, J = 16.8 Hz, 1H), 3.81(s. 3H), 3.79 (d, J = 16.8 Hz, 1H), 2.14 (m, 1H), 1.72 (m, 1H), 1.60 (m, 1H), 0.95 (d, J = 6.3 Hz, 3H), 0.88 (d, J = 6.3 Hz, 3H).

### Example 3

N-[2-[4-Cyano-2-[4-methyl-2-(1-naphthyl)pentanoylamino]phenyl]metnylbenzoyl]-phenylsulfonamide

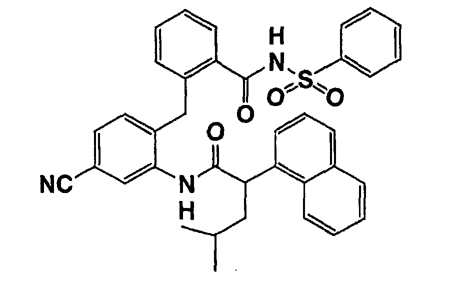

To a solution of the compound prepared in example 2 (200 mg) in methylene chloride (1.5 ml) was added 1-(3-dimethylaminopropyl)-3-ethylcarbodiimide (121 mg) and 4-dimethylaminopyridine (15.4 mg) and the mixture was stirred overnight at room temperature. The reaction mixture was diluted with ethyl acetate, and the diluted solution was washed with 1N hydrochloric acid, water and a saturated aqueous solution of sodium chloride, dried over anhydrous magnesium sulfate and was concentrated. The residue was purified by column chromatography on silica gel (ethyl acetate : hexane = 3 : 2) to give the title compound (27 mg) having the following physical data.
TLC: Rf 0.42 (ethyl acetate : hexane = 2:1);
NMR (300 MHz, d₆-DMSO) : δ 9.90 (bs, 1H), 8.32 (d, J= 8.4 Hz, 1H), 7.98-7.75 (m, 5H), 7.62-7.28 (m, 10H), 7.21-7.09 (m, 2H), 6.91 (bs, 1H), 6.82 (d, J = 6.9 Hz, 1H), 4.69 (m, 1H), 4.00 (d, J = 15.3 Hz, 1H), 3.92 (d, J = 15.3 Hz, 1H), 1.95 (m, 1H), 1.59-1.42 (m, 2H), 0.94 (d, J = 6.0 Hz, 3H), 0.81 (d, J = 6.0 Hz, 3H).

### Example 4

2-[2-[2-Hydroxy-2-(1-naphthyl)acetylamino]phenyl]methylbenzoic acid

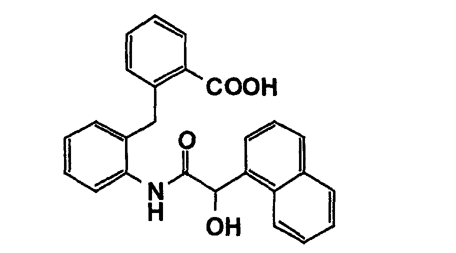

To a solution of the compound prepared in example 2(25) (262 mg) in methanol (3.0 ml) was added 4N hydrogen chloride in dioxane (2 ml) and the mixture was stirred overnight at room temperature. The reaction mixture was concentrated. The residue was purified by column chromatography on silica gel (n-hexane : ethyl acetate = 4 : 1) to give the title compound (141 mg) having the following physical data.
TLC: Rf 0.59 (ethyl acetate);
NMR (300 MHz, d₆-DMSO) : δ 9.60 (brs, 1H), 8.29 (m, 1H), 7.95-7.83 (m, 3H), 7.64 (d, J = 8.1 Hz, 1H), 7.53-7.33 (m, 6H), 7.18 (m, 1H), 7.10-7.02 (m, 2H), 6.91 (d, J = 7.8 Hz, 1H), 6.73 (brs, 1H), 5.78 (s, 1H),4.44 (d, J = 16.2 Hz, 1H), 4.33 (d, J = 16.2 Hz, 1H).

### Reference Example 3

2-(4-Hydroxy-2-nitrobenzyl)benzoic acid methyl ester

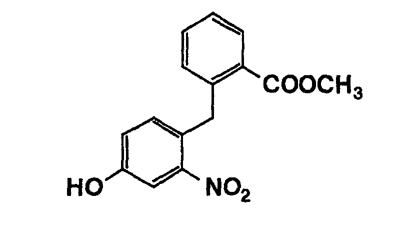

To a solution of 2-(4-methoxy-2-nitrobenzyl)benzoic acid methyl ester (1.84 g), which prepared by the same procedure as described in example 1 using 1-iodo-4-methoxy-2-nitrobenzene, in methylene chloride (20 ml) was added 1M solution of boron tribromide in methylene chloride (18.3 ml) at -78 °C and the mixture was stirred for 8 hours at room temperature. To the reaction mixture was added water and then extracted with ehter. The organic layer was washed with a saturated aqueous solution of sodium chloride, dried over anhydrous sodium sulfate and the concentrated. The residue was dissolved in methanol. The mixture was added sulfuric acid (3 ml) and stirred overnight at 60 °C. The reaction mixture was diluted with ethyl acetate, washed with water and a saturated aqueous solution of sodium chloride, dried over anhydrous sodium sulfate, and then concentrated. The residue was purified by column chromatography on silica gel (n-hexane : ethyl acetate = 3 : 1) to give the title compound (1.15 g) having the following physical data.
TLC: Rf 0.34 (chloroform : methanol = 10 : 1);
NMR (300 MHz, 0D018): δ 7.97 (dd, J = 7.8, 1.5 Hz, 1H), 7.48-7.42(m, 2H), 7.33 (m, 1H), 7.12 (d, J = 8.4 Hz, 1H), 6.95-6.86 (m, 2H), 5.63 (s, 1H), 4.57 (s, 2H), 3.80 (s, 3H).

### Reference Example 4

2-(4-Methoxymethoxy-2-nitrobenzyl)benzoic acid methyl ester

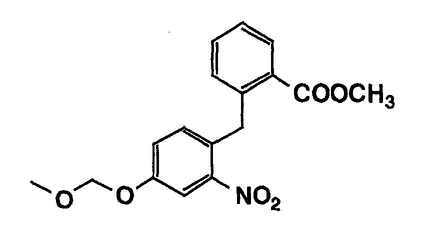

To a solution of the compound prepared in reference example 3 (870 mg) in methylene chloride (15 ml) was added N,N-diisopropylethylamine (1.04 ml) and methoxymethoxychloride (364 µl) and the mixture was stirred for 5 hours. The reaction mixture was diluted with ethyl acetate. The diluted solution was washed with a saturated aqueous solution of sodium bicarbonate, water and a saturated aqueous solution of sodium chloride, dried over anhydrous sodium sulfate, and was concentrated to give the title compound (1.00 g) having the following physical data.
TLC: Rf 0.88 (chloroform : methanol = 10 : 1);
NMR (300 MHz, CDCl₃): δ 7.96 (dd, J = 7.8, 1.5 Hz, 1H), 7.46-7.32(m, 3H), 7.09-6.92 (m, 3H), 5.15 (s, 2H), 3.78 (s, 3H), 3.45 (s, 3H).

### Reference Example 5

2-(4-Methoxymethoxy-2-aminobenzyl)benzoic acid methyl ester

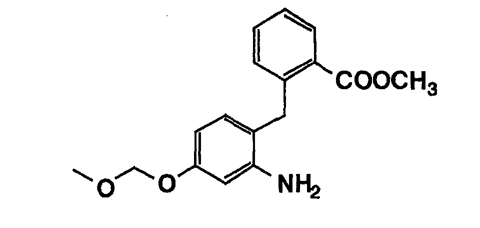

To a solution of the compound prepared in reference example 4 in methanol was added 10% palladium on carbon (100 mg) and the mixture was stirred for 4 hours under an atmosphere of hydrogen. The reaction mixture was filtered over celite (brand name). The filtrate was concentrated to give the title compound having the following physical data.
TLC: Rf 0.53 (chloroform : methanol = 10 : 1).

### Example 5

2-[4-Hydroxy-2-[4-methyl-2-(1-naphthyl)pentanoylamino]phenyl]methylbenzoic acid

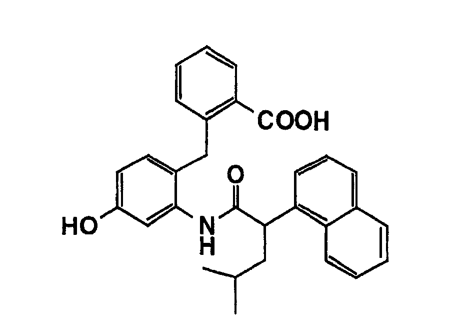

By the same procedure as described in Example 1 -> Example 4 -> Example 2 using the compound prepared in reference example 5 and 4-methyl-2-(1-naphthyl)valeryl chloride, the compound having the following physical data was given.
TLC: Rf 0.46 (chloroform : methanol = 10 : 1);
NMR (300 MHz, d₆-DMSO): δ 9.46 (s, 1H), 9.21 (s, 1H), 8.28 (d, J = 8.1 Hz, 1H), 7.90 (d, J = 7.5 Hz, 1H), 7.77 (d, J = 8.4 Hz, 1H), 7.69 (m, 1H), 7.60-7.37 (m, 4H), 7.25-7.14 (m, 2H), 6.91 (d, J = 2.7 Hz, 1H), 6.80-6.71 (m, 2H), 6.46 (dd, J = 8.1, 2.7 Hz, 1H), 4.55 (m, 1H), 4.20 (d, J = 16.8 Hz, 1H), 4.07 (d, J = 16.8 Hz, 1H), 1.90 (m, 1H), 1.51-1.30 (m, 2H), 0.90 (d, J = 6.0 Hz, 3H), 0.78 (d, J = 6.0 Hz, 3H).

### Reference Example 6

2-(2-Methoxycarbonylbenzyl)-5-nitrobenzoic acid

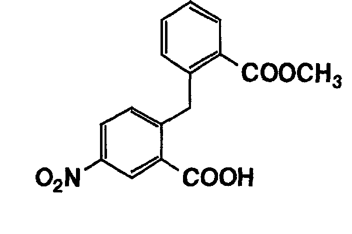

A solution of 2-(2-methoxycarbonylbenzyl)-4-nitrobenzoic acid t-butyl ester (4.5 g), which prepared by the same procedure as described in example 1 using 5-nitro-2-iodobenzoic acid t-butyl ester, in trifluoroacetic acid (5 ml) and anisole (2 ml) was stirred for 3 hours at 50 °C. The solvent was removed from the reaction solution to give a crude crystal. The crude crystal was recrystallized from n-hexane / ether to give the title compound (3.3 g) having the following physical data.
TLC: Rf 0.30 (chloroform : methanol = 10 : 1);
NMR (300 MHz, CDCl₃) : δ 8.93 (d, J =2.4 Hz, 1H), 8.21 (dd, J = 8.4, 2.4 Hz, 1H), 8.03 (dd, J = 7.8, 1.5 Hz, 1H), 7.50 (dt, J = 7.7, 1.5 Hz, 1H), 7.38 (dt, J = 7.7, 1.2 Hz, 1H), 7.16 (dd, J = 7.7, 1.2 Hz, 1H), 7.12 (d, J = 8.4 Hz, 1H), 4.89 (s, 2H), 3.80 (s, 3H).

### Reference Example 7

2-(2-t-Butoxycarbonylamino-4-nitrobenzyl)benzoic acid methyl ester

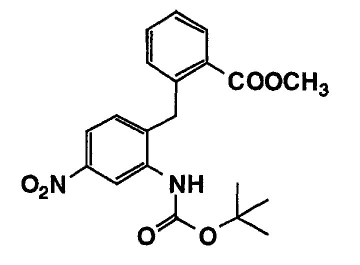

To a solution of the compound prepared in reference example 6 (3.2 g) in t-butanol (34 ml) was added triethylamine (1.4 ml) and diphenylphosphoryl azide (2.3 ml) under an atmosphere of argon, and the mixture was refluxed for 3 hours. The reaction mixture was filtered and the filtrate was concentrated. The residue was purified by column chromatography on silica gel (n-hexane : ethyl acetate = 8 : 1) to give the title compound (3.4 g) having the following physical data.
TLC: Rf 0.44 (n-hexane : ethyl acetate = 3 : 1);
NMR (300 MHz, CDCl₃: δ 8.82 (d, J = 1.5 Hz, 1H), 7.98 (dd, J = 8.4, 1.5 Hz, 1H), 7.84 (dd, J = 7.5, 2.4 Hz, 1H), 7.76 (bs, 1H), 7.45 (dt, J = 7.5, 1.8 Hz, 1H), 7.34 (dt, J = 1.2, 7.5 Hz, 1H), 7.22 (d, J = 8.4 Hz, 1H), 7.16 (dd, J = 7.5, 1.2 Hz, 1H), 4.39 (s, 2H), 3.91 (s, 3H), 1.45 (s, 9H).

### Reference Example 8

2-[4-Ammo-2-[N-[4-methyl-2-(1-naphthyl)pentanoyl] -N-t-butoxycarbonylamino]phenyl]methylbenzoic acid methyl ester

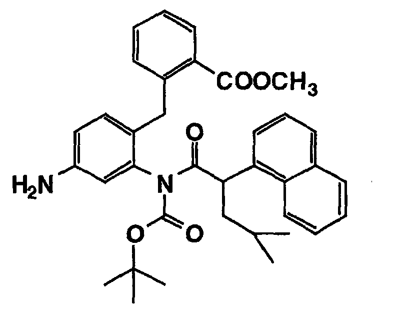

By the same procedure as described in example 1 -> reference example 5 using the compound prepared in reference example 7 (2.65 g) and 4-methyl-2-(1-naphthyl)valeryl chloride (2.68 g), the title compound (3.4 g) having the following physical data was given.
TLC: Rf 0.32 (n-hexane : ethyl acetate = 3 : 1);
NMR (300 MHz, CDCl₃) : δ 7.99(m, 1H), 7.97-7.18 (m, 8H), 7.10 (m, 1H), 6.64 (m, 1H), 6.45 (m, 1H), 6.00 (m, 1H), 5.61 (m, 1H), 4.10 (d, J = 16.5 Hz, 1H), 3.94 (d, J = 16.5 Hz, 1H), 3.80 (s, 3H), 2.19 (m, 1H), 1.74-1.41 (m, 2H), 1.22 (s, 9H), 0.98 (d, J = 6.3 Hz, 3H), 0.89 (d, J = 6.3 Hz, 3H).

### Example 6

2-[4-Dimethylamino-2-[4-methyl-2-(1-naphthyl)pentanoylamino]phenyl]methylbenzoic acid

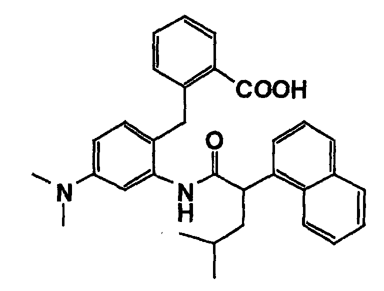

The compound prepared in reference example 8 (250 mg), formaldehyde (35% solution in water, 0.37 ml) and acetic acid (32 µl) were dissolved in acetonitrile (1.2 ml). To the mixture was added sodium cyanoborohydride (81 mg) and the mixture was stirred for 15 minutes at room temperature. The reaction mixture was extracted with ether. The organic layer was washed with water and a saturated aqueous solution of sodium chloride, dried over anhydrous sodium sulfate, and then concentrared. The residue was dissolved in methanol. To the mixture was added 4N hydrogen chloride in dioxane (3 ml). The mixture was stirred for 1 hour at room temperature and then concentrated. The residue was dissolved in methanol (3 ml) and THF (3 ml). To the mixture was added 2N aqueous solution of sodium hydroxide (3 ml), then the mixture was stirred overnight. The reaction mixture was diluted with ethyl acetate, and the diluted solution was washed with 1N hydrochloric acid, water and a saturated aqueous solution of sodium chloride, dried over anhydrous sodium sulfate, and concentrated. The residue was purified by column chromatography on silica gel (chloroform : methanol = 40 : 1) to give the title compound (85 mg) having the following physical data
TLC: Rf 0.32 (chloroform : methanol = 10 : 1);
NMR (300 MHz, CDCl₃) : δ 8.03 (d, J = 8.1 Hz, 1H), 7.85-7.07 (m, 11H), 6.90 (d, J = 8.4 Hz, 1H), 6.78 (d, J = 7.2 Hz, 1H), 6.44 (d, J = 7.2 Hz, 1H), 4.32 (t, J = 7.2 Hz, 1H), 3.97 (d, J = 17.1 Hz, 1H), 3.78 (d, J = 17.1 Hz, 1H), 2.94 (s, 6H), 2.13 (m, 1H), 1.73 (m, 1H), 1.57 (m, 1H), 0.93 (d, J = 6.3 Hz, 3H), 0.87 (d, J = 6.3 Hz, 3H).

### Reference Example 9

2-(2-Aminobenzyl)benzylalcohol

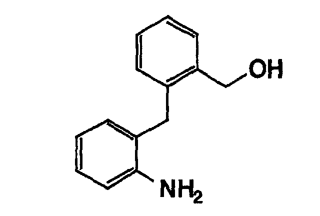

To a solution of 2-(2-aminobenzyl)benzoic acid methyl ester (400 mg), which prepared by the same procedure as described in reference example 1 => reference example 2 using 1-nitro-2-iodobenzene, in THF (5.5 ml) was added lithium aluminium hydride (157 mg) at -0 °C and the mixture was stirred for 2 hours at room temperature. To the reaction solution was added a saturated aqueous solution of sodium sulfate, and the mixture was stirred for 1 hour to precipitate salts. The solution was filtered over celite (brand name) and the filtrate was concentrated. The crude crystal was recrystallized from ethyl acetate / n-hexane to give the title compound (291 mg) having the following physical data.
TLC: Rf 0.29 (n-hexane : ethyl acetate = 1 : 1);
NMR (200 MHz, CDCl₃ : δ 7.40 (m, 1H), 7.28-7.22 (m, 2H), 7.14-7.04 (m, 2H), 6.95 (m, 1H), 6.76 (m, 1H), 6.69 (m, 1H), 4.74 (s, 2H), 3.98 (s, 2H).

### Reference Example 10

2-[2-(t-Butyldimethylsilyloxymethyl)benzyl]aniline

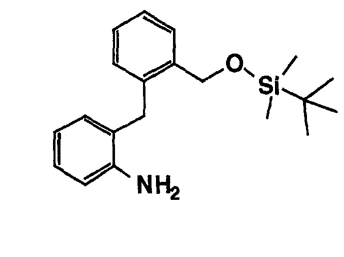

To a solution of the compound prepared in reference example 9 (290 mg) in DMF was added t-butyldimethylsilyl chloride (225 mg) and imidazole (185 mg), and the mixture was stirred for 2 hours at room temperature. The reaction solution was diluted with ethyl acetate and the diluted solution was washed with 1N hydrochloric acid, a saturated aqueous solution of sodium bicarbonate and a saturated aqueous solution of sodium chloride, dried over anhydrous sodium sulfate, and concentrated. The residue was purified by column chromatography on silica gel (n-hexane : ethyl acetate = 6 : 1) to give the title compound (400 mg) having the following physical data.
TLC: Rf 0.89 (n-hexane : ethyl acetate = 1:1);
NMR (300 MHz, CDCl₃) : δ 7.42 (dd, J = 7.5, 1.2 Hz, 1H), 7.24-7.06 (m, 3H), 6.98 (d, J = 7.8 Hz, 2H), 6.73 (dt, J = 7.5, 1.2 Hz, 1H), 6.68 (dd, J = 8.1, 1.2 Hz, 1H), 4.76 (s, 2H), 3.91 (s, 2H), 0.94 (s, 9H), 0.01 (s, 6H).

### Example 7

2-[2-[2-(1-Naphthyl)propanoylamino]phenyl]methylbenzylalcohol

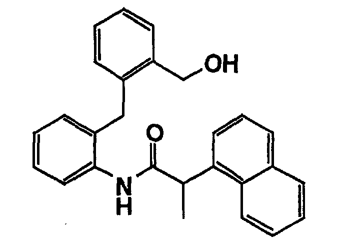

To a solution of the compound prepared in reference example 10 (400 mg) in methylene chloride (6 ml) was added 2-(1-naphthyl)propionyl chloride (400 mg) and pyridine (198 µl), and the mixture was stirred overnight at room temperature. The reaction solution was diluted with ethyl acetate, then the diluted solution was washed with 1N hydrochloric acid, a saturated aqueous solution of sodium bicarbonate and a saturated aqueous solution of sodium chloride, dried over anhydrous sodium sulfate, and then concentrated. The residue was purified by column chromatography on silica gel (n-hexane : ethyl acetate = 6 : 1) to give an oil.

To a solution of the oil in THF (3.6 ml) was added 1M tetrabutylammonium fluoride (2.19 ml; THF solution), and the mixture was stirred overnight at room temperature. The reaction mixture was diluted with ethyl acetate, and the diluted solution was washed with 1N hydrochloric acid, water and a saturated aqueous solution of sodium chloride, dried over anhydrous sodium sulfate, and then concentrated. The residue was purified by column chromatography on silica gel (n-hexane : ethyl acetate = 1 : 1) to give the title compound (400 mg) having the following physical data.
TLC: Rf 0.48 (n-hexane : ethyl acetate = 1:1);
NMR (300 MHz, CDCl₃) : δ 8.04-7.70 (m, 3H), 7.54-7.46 (m, 2H), 7.36-6.94 (m, 10H), 6.66 (d, J=7.8Hz, 1H), 4.34 (q, J = 7.2 Hz, 1H), 4.25 (bs, 2H), 3.55 (s, 2H), 1.64 (d, J=7.2Hz, 3H).

### Formulation example 1

The following components were admixed in conventional meth od and punched out to obtain 100 tablets each containing 5 mg of ac tive ingredient.
- 2-[2-[2-(4-benzyloxyphenyl)propanoylamino]phenyl]methylbenzoic acid 500mg
- Carboxymethylcellulose calcium (disintegrating agent) 200mg
- Magnesium stearate (lubricating agent) 100mg
- Microcrystalline cellulose 9.2g

## Claims

1. A pharmaceutical composition having an activity of Prostaglandin E₂ receptor subtype EP₃ antagonist, which comprises a benzoic acid derivative of formula (I) 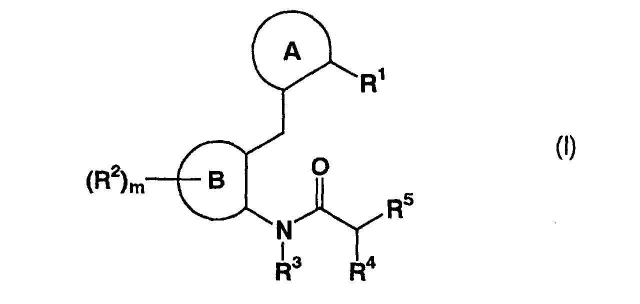 wherein R¹ is COOH, COOR⁶, CH₂OH, CONHSO₂R⁷ or CONR⁸R⁹,
R⁶ is C1-6 alkyl, (C1-4 alkylene)-R¹⁶,
R⁷ is (1) C1-4 alkyl, or (2) substituted by 1-2 of substitutes selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituents or unsubstituted carbocyclic ring or heterocyclic ring,
R⁸ and R⁹ each independently, is hydrogen or C1-4 alkyl,
R¹⁶ is hydroxy, C1-4 alkoxy, COOH, C1-4 alkoxycarbonyl, CONR⁸R⁹,
A is C5-6 mono-carbocyclic ring or 5-6 membered mono-heterocyclic ring containing at least one of nitrogen atom, oxygen atom and sulfur atom, the mono-carbocyclic ring or mono-heterocyclic ring may be substituted by 1-2 of substitutes selected from C1-4 alkyl, C1-4 alkoxy, halogen atom, CF₃, cyano and nitro,
R² is C1-6 alkyl, C2-6 alkenyl, C2-6 alkynyl, C1-6 alkoxy, halogen atom, CF₃, cyano, hydroxy, nitro, NR¹⁰R¹¹, CONR¹⁰R¹¹, SO₂NR¹⁰R¹¹, or -S(O)x-(C1-6)alkyl,
m is 0, 1 or 2, when m is 2, then two R² may be same or difference,
R¹⁰ and R¹¹ each independently, hydrogen or C1-4 alkyl,
x is 0, 1 or 2,
B ring is C5-7 mono-carbocyclic ring or 5-7 membered mono-heterocyclic ring containing at least one of nitrogen atom, oxygen atom and sulfur atom,
R³ is hydrogen or C1-4 alkyl,
R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
R⁵ is substituted by 1-2 of R¹² or unsubstituted C5-10 mono- or bi-carbocyclic ring or 5-10 membered mono- or bi-heterocyclic ring containing at least one of nitrogen atom, oxygen atom and sulfur atom,
R¹² is C1-6 alkyl, C1-6 alkoxy, halogen atom, CF₃, cyano, C1-4 alkoxy(C1-4)alkyl, phenyl, phenyl(C1-6)alkyl, -(C1-4 alkylene)_{y}-G-(C1-8 alkylene)ₓ-R¹³, benzoyl or thiophenecarbonyl and two R¹² may be same or difference,
y is 0 or 1,
z is 0 or 1,
R¹³ is phenyl or pyridyl,
G is oxygen atom, S(O)ₜ or NR¹⁴,
t is 0, 1 or 2,
R¹⁴ is hydrogen, C1-4 alkyl or acetyl;
or non-toxic salts thereof.

2. A pharmaceutical composition according to the claim 1 selected from
(1) 2-[4-Cyano-2-[4-methyl-2-(1-naphthyl)pentanoylamino]phenyl]methylbenzoic acid,
(2) 2-[2-[2-(4-Benzyloxyphenyl)propanoylamino]phenyl]methylbenzoic acid,
(3) 2-[2-[2-(3-Benzyloxyphenyl)propanoylamino]phenyl]methylbenzoic acid,
(4) 2-[2-[2-(1-Naphthyl)butanoylamino]phenyl]methylbenzoic acid,
(5) 2-[2-[2-(1-Naphthyl)pentanoylamino]phenyl]methylbenzoic acid,
(6) 2-[2-[3-Methyl-2-(1-naphthyl)butanoylamino]phenyl]methylbenzoic acid,
(7) 2-[2-[2-(1,1'-Biphenyl-2-yl)propanoylamino]phenyl]methylbenzoic acid,
(8) 2-[2-[2-(1,1'-Biphenyl-3-yl)propanoylamino]phenyl]methylbenzoic acid,
(9) 2-[2-[2-(4-Phenoxyphenyl)propanoylamino]phenyl]methylbenzoic acid,
(10) 2-[2-[2-[4-(2-Phenylethoxy)phenyl]propanoylamino]phenyl]methylbenzoic acid,
(11) 2-[2-[2-[4-(3-Phenylpropoxy)phenyl]propanoylamino]phenyl]methylbenzoic acid,
(12) 2-[2-[2-Methoxy-2-(1-naphthyl)acetylamino]phenyl]methylbenzoic acid,
(13) 2-[2-[2-(4-Isobutylphenyl)propanoylamino]phenyl]methylbenzoic acid,
(14) 2-[2-[4-Methyl-2-(1-naphthyl)pentanoylamino]phenyl]methylbenzoic acid,
(15) 2-[2-[2-(1-N aphthyl)hexanoylamino]phenyl]methylbenzoic acid,
(16) 2-[2-[2-[4-(4-Phenylbutoxy)phenyl]prop anoylamino]phenyl]methylbenzoic acid,
(17) 2-[2-[2(R)-(1-Naphthyl)propanoylamino]phenyl]methylbenzoic acid,
(18) 2-[2-[2(S)-(1-Naphthyl)propanoylamino]phenyl]methylbenzoic acid,
(19) 2-[2-[2-Ethoxy-2-(1-naphthyl)acetylamino]phenyl]methylbenzoic acid,
(20) 2-[2-[2-(1-Naphthyl)heptanoylamino]phenyl]methylbenzoic acid,
(21) 2-[2-[4,4-Dimethyl-2-(1-naphthyl)pentanoylamino]phenyl]methylbenzoic acid,
(22) 2-[2-[2-[4-(3-Phenylpropyl)phenyl]prop anoylamino]phenyl]methylbenzoic acid,
(23) 2-[2-[2-(Quinolin-5-yl)propanoylamino]phenyl]methylbenzoic acid,
(24) 2-[2-[2-[4-[2-(3-Pyridyl)ethoxy]phenyl]prop anoylamino]phenyl]methylbenzoic acid,
(25) 2-[2-[2-[4-(2-Phenoxyethyl)phenyl]propanoylamino]phenyl]methylbenzoic acid,
(26) 2-[2-[4-Methoxy-2-(1-naphthyl)butanoylamino]phenyl]methylbenzoic acid,
(27) 2-[2-[5-Methyl-2-(1-naphthyl)hexanoylamino]phenyl]methylbenzoic acid,
(28) 5-[2-[2-(1-Naphthyl)propanoylamino]phenyl]methyloxazole-4-carboxylic acid,
(29) 2-[2-[2-[4-(2-Phenylethylthio)phenyl]propanoylamino]phenyl]methylbenzoic acid,
(30) 2-[2-[3-Methoxy-2-(1-naphthyl)propanoylamino]phenyl]methylbenzoic acid,
(31) 2-[4-Ethoxy-2-[4-methyl-2-(1-naphthyl)p entanoylamino]phenyl]methylbenzoic acid,
(32) 2-[4-Isopropyloxy-2-[4-methyl-2-(1-1-naphthyl)pentanoylamino]phenyllmethylbenzoic acid,
(33) 5-[2-[4-Methyl-2-(1-naphthyl)pentanoylamino]phenyl]methyloxazole-4-carboxylic acid,
(34) 2-[4-Methoxy-2-[4-methyl-2-(1-naphthyl)pentanoylamino]phenyl]methylbenzoic acid,
(35) 2-[2-[2-[4-(2-Thienyl)carbonylphenyl]propanoylamino]phenyl]methylbenzoic acid,
(36) 2-[2-[3-Cyclopropyl-2-(1-naphthyl)propanoylamino]phenyl]methylbenzoic acid,
(37) 2-[2-[2-Cyclopropyl-2-(1-naphthyl)acetylamino]phenyl]methylbenzoic acid,
(38) 2-[2-[2-(4-Benzoylphenyl)propanoylamino]phenyl]methylbenzoic acid,
(39) 2-[2-[3-Phenyl-2-(1-naphthyl)propanoylamino]phenyl]methylbenzoic acid,
(40) 2-[2-[2-Methoxymethoxy-2-(1-naphthyl)acetylamino]phenyl]methylbenzoic acid,
(41) 2-[2-[3-Cyclobutyl-2-(1-naphthyl)propanoylamino]phenyl]methylbenzoic acid,
(42) 2-[2-[2-(4-Benzyloxymethylphenyl)propanoylamino]phenyl]methylbenzoic acid,
(43) 2-[2-[2-[4-[N-Methyl-N-(2-phenylethyl)amino]phenyl]propanoylamino]phenyl]methylbenzoic acid,
(44) 2-[2-[2-[4-[N-Acetyl-N-(2-phenylethyl)amino]phenyl]propanoylamino]phenyl]methylbenzoic acid,
(45) 2-[2-[2-[4-[N-(2-Phenylethyl)amino]phenyl]propanoylamino]phenyl]methylbenzoic acid,
(46) 2-[2-[2-[4-(2-Phenylethylsulfinyl)phenyl]propanoylamino]phenyl]methylbenzoic acid,
(47) 2-[2-[2-[4-(2-Phenylethylsulfonyl)phenyl]propanoylamino]phenyl]methylbenzoic acid,
(48) 2-[2-[2-(1-Naphthyl)-4-pentenoylamino]phenyl]methylbenzoic acid,
(49) 2-[2-[2-(1-Naphthyl)-4-hexenoylamino]phenyl]methylbenzoic acid,
(50) 2-[2-[4-Ethyl-2-(1-naphthyl)hexanoylamino]phenyl]methylbenzoic acid,
(51) 2-[2-[2-(1-Naphthyl)-4-pentynoylamino]phenyl]methylbenzoic acid,
(52) 2-[4-Cyano-2-[3-cyclopropyl-2-(1-1-naphthyl)propanoylamino]phenyl]methylbenzoic acid,
(53) 2-[2-[5-Methyl-2-(1-naphthyl)-4-hexenoylamino]phenyl]methylbenzoic acid,
(54) N-[2-[4-Cyano-2-[4-methyl-2-(1-naphthyl)pentanoylamino]phenyl]metnylbenzoyl]-phenylsulfonamide,
(55) 2-[2-[2-Hydroxy-2-(1-naphthyl)acetylamino]phenyl]methylbenzoic acid,
(56) 2-[4-Hydroxy-2-[4-methyl-2-(1-naphthyl)pentanoylamino]phenyl]methylbenzoic acid,
(57) 2-[4-Dimethylamino-2-[4-methyl-2-(1-naphthyl)pentanoylamino]phenyl]methylbenzoic acid and
(58) 2-[2-[2-(1-Naphthyl)propanoylamino]phenyl]methylbenzylalcohol or non-toxic salts thereof.

3. A benzoic acid derivative of formula (I) according to claim 1 or non-toxic salts thereof for use in the prevention and / or treatment of pain such as pain such as cancerous pain, fractural pain, pain following surgical and dental procedures; allodynia, hyperalgesia, pruritus, urticaria, atopic dermatitis, contact dermatitis, allergic conjunctivitis, various symptoms by treating with dialysis, asthma, rhinitis, sneeze, urinary frequency, neurogenic bladder, urinary disturbance, ejaculatory failure, defervescence, systemic inflammatory response syndrome, learning disturbance, Alzheimer' s disease, cancer such as formulation of cancer, growth of cancer and metastasis of cancer; retinopathy, patch of red, scald, burn, burn by steroid, renal failure, nephropathy, acute nephritis, chronic nephritis, abnormal blood levels of electrolytes, threatened premature delivery, abortion threatened, hypermenorrhea, dysmenorrhea, uterine fibroids, premenstrual syndrome, reproductive disorder, stress, anxiety disorders, depression, psychosomatic disorder, mental disorder, thrombosis, embolism, transient ischemia attack, cerebral infarction, atheroma, organ transplant, myocardial infarction, cardiac failure, hypertension, arteriosclerosis, circulatory failure and circulatory failure induced ulcer, neuropathies, vascular dementia, edema, various arthritis, rheumatism, diarrhea, constipation, disorder of bilious excretion, ulcerative colitis, Crohn's disease.

4. A benzoic acid derivative of formula (I-A) 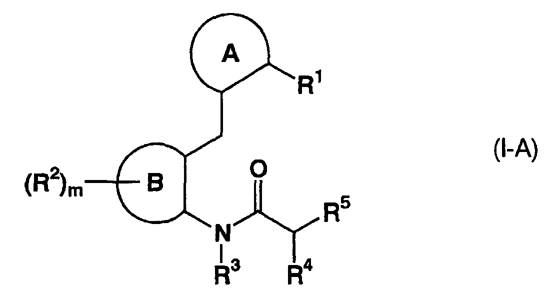 wherein R¹ is COOH, COOR⁶, CH₂OH, CONHSO₂R⁷ or CONR⁸R⁹,
R⁶ is C1-6 alkyl, (C1-4 alkylene)-R¹⁶,
R⁷ is (1) C1-4 alkyl, or (2) substituted by 1-2 of substitutes selected form C1-4 alkyl, C1-4 alkoxy and halogen atom or unsubstituted (2-1) C6-12 mono- or bi-carbocyclic ring or (2-2) 5-15 membered mono- or bi-heterocyclic ring containing at least one of hetero atom selected from nitrogen atom, oxygen atom and sulfur atom, or (3) C1-4 alkyl substituted by the above substituents or unsubstituted carbocyclic ring or heterocyclic ring,
R⁸ and R⁹ each independently, is hydrogen or C1-4 alkyl,
R¹⁶ is hydroxy, C1-4 alkoxy, COOH, C1-4 alkoxycarbonyl, CONR⁸R⁹,
A is C5-6 mono-carbocyclic ring or 5-6 membered mono-heterocyclic ring containing at least one of nitrogen atom, oxygen atom and sulfur atom, the mono-carbocyclic ring or mono-heterocyclic ring may be substituted by 1-2 of substitutes selected from C1-4 alkyl, C1-4 alkoxy, halogen atom, CF₃, cyano and nitro,
R² is C1-6 alkyl, C2-6 alkenyl, C2-6 alkynyl, C1-6 alkoxy, halogen atom, CF₃, cyano, hydroxy, nitro, NR¹⁰R¹¹, CONR¹⁰R¹¹, SO₂NR¹⁰R¹¹, or -S(O)ₓ-(C 1-6)alkyl,
m is 0, 1 or 2, when m is 2, then two R² may be same or difference,
R¹⁰ and R¹¹ each independently, hydrogen or C1-4 alkyl,
x is 0, 1 or 2,
B ring is C5-7 mono-carbocyclic ring or 5-7 membered mono-heterocyclic ring containing at least one of nitrogen atom, oxygen atom and sulfur atom,
R³ is hydrogen or C 1-4 alkyl,
R⁴ is (1) C1-8 alkyl, (2) C2-8 alkenyl, (3) C2-8 alkynyl, (4) C3-6 cycloalkyl, (5) hydroxy, (6) C1-6 alkoxy, (7) C1-4 alkoxy(C1-4)alkoxy, or (8) C1-8 alkyl substituted by 1-2 of substitutes selected from halogen atom, hydroxy, C1-6 alkoxy, C1-4 alkoxy(C1-4)alkoxy, phenyl and C3-6 cycloalkyl, R⁶ is substituted by 1-2 of R¹² or unsubstituted C5-10 mono- or bi-carbocyclic ring or 5-10 membered mono- or bi-heterocyclic ring containing at least one of nitrogen atom, oxygen atom and sulfur atom,
R¹² is C1-6 alkyl, C1-6 alkoxy, halogen atom, CF₃, cyano, C1-4 alkoxy(C1-4)alkyl, phenyl, phenyl(C1-6)alkyl, -(C 1-4 alkylene)_{y}-G-(C1-8 alkylene)ₓ-R¹³, benzoyl or thiophenecarbonyl and two R¹² may be same or difference,
y is 0 or 1,
z is 0 or 1,
R¹³ is phenyl or pyridyl,
G is oxygen atom, S(O)ₜ or NR¹⁴,
t is 0, 1 or 2,
R¹⁴ is hydrogen, C1-4 alkyl or acetyl;
with the proviso, at least one of the R¹, R², R⁴ and R¹² is as follows,
R¹ is COO-(C1-4 alkylene)-R¹⁶, CH₂OH or CONHSO₂R⁷,
R² is C2-6 alkenyl, C2-6 alkynyl, hydroxy, NR¹⁰R¹¹, CONR¹⁰R¹¹,-SO₂NR¹⁰R¹¹, or -S(O)ₓ-(C1-4)alkyl,
R⁴ is (1) C6-8 alkyl, (2) C2-8 alkenyl, (3) C2-8 alkynyl, (4) C3-6 cycloalkyl, (5) hydroxy, (6) C1-4 alkoxy(C1-4)alkoxy, or (7) C1-8 alkyl substituted by 1-2 of substitutes selected from halogen atom, hydroxy, C1-6 alkoxy, C1-4 alkoxy(C1-4)alkoxy, phenyl and C3-6 cycloalkyl,
R¹² is -(C1-4 alkylene)-G-(C1-8 alkylene)-R¹³, -(C1-4 alkylene)-G₁-R¹³, -G₁-(C1-8 alkylene)-R¹³, benzoyl or thiophenecarbonyl,
G¹ is oxygen atom, S(O)ₜ or NR¹⁴;
or non-toxic salts.

5. A benzoic acid derivative according to the claim 4 selected from
(1) 2-[2-[2-[4-(2-Thienyl)carbonylphenyl]propanoylamino]phenyl]methylbenzoic acid,
(2) 2-[2-[3-Cyclopropyl-2-(1-naphthyl)propanoylamino]phenyl]methylbenzoic acid,
(3) 2-[2-[2-Cyclopropyl-2-(1-naphthyl)acetylamino]phenyl]methylbenzoic acid,
(4) 2-[2-[2-(4-Benzoylphenyl)propanoylamino]phenyl]methylbenzoic acid,
(5) 2-[2-[3-Phenyl-2-(1-naphthyl)propanoylamino]phenyl]methylbenzoic acid,
(6) 2-[2-[2-Methoxymethoxy-2-(1-naphthyl)acetylamino]phenyl]methylbenzoic acid,
(7) 2-[2-[3-Cyclobutyl-2-(1-naphthyl)prop anoylamino]phenyl]methylbenzoic acid,
(8) 2-[2-[2-(4-Benzyloxymethylphenyl)propanoylamino]phenyl]methylbenzoic acid,
(9) 2-[2-[2-[4-[N-Methyl-N-(2-phenylethyl)amino]phenyl]propanoylamino]phenyl]methylbenzoic acid,
(10) 2-[2-[2-[4-[N-Acetyl-N-(2-phenylethyl)amino]phenyl]propanoylamino]phenyl]methylbenzoic acid,
(11) 2-[2-[2-[4-[N-(2-Phenylethyl)aino]phenyl]propanoylamino]phenyl]methylbenzoic acid,
(12) 2-[2-[2-[4-(2-Phenylethylsulfinyl)phenyl]propanoylamino]phenyl]methylbenzoic acid,
(13) 2-[2-[2-[4-(2-Phenylethylsulfonyl)phenyl]propanoylamino]phenyl]methylbenzoic acid,
(14) 2-[2-[2-(1-Naphthyl)-4-pentenoylamino]phenyl]methylbenzoic acid,
(15) 2-[2-[2-(1-Naphthyl)-4-hexenoylamino]phenyl]methylbenzoic acid,
(16) 2-[2-[4-Ethyl-2-(1-naphthyl)hexanoylamino]phenyl]methylbenzoic acid,
(17) 2-[2-[2-(1-Naphthyl)-4-pentynoylamino]phenyl]methylbenzoic acid,
(18) 2-[4-Cyano-2-[3-cyclopropyl-2-(1-naphthyl)propanoylamino]phenyl]methylbenzoic acid,
(19) 2-[2-[5-Methyl-2-(1-naphthyl)-4-hexenoylamino]phenyl]methylbenzoic acid,
(20) N-[2-[4-Cyano-2-[4-methyl-2-(1-naphthyl)pentanoylamino]phenyl]metnylbenzoyl]-phenylsulfonamide,
(21) 2-[2-[2-Hydroxy-2-(1-naphthyl)acetylamino]phenyl]methylbenzoic acid,
(22) 2-[4-Hydroxy-2-[4-methyl-2-(1-naphthyl)pentanoylamino]phenyl]methylbenzoic acid,
(23) 2-[4-Dimethylamino-2-[4-methyl-2-(1-naphthyl)pentanoylamino]phenyl]methylbenzoic acid and
(24) 2-[2-[2-(1-Naphthyl)propanoylamino]phenyl]methylbenzylalcohol or non-toxic salts thereof.

6. A pharmaceutical composition having an activity of Prostaglandin E₂ receptor subtype EP₄ antagonist, which comprises a benzoic acid derivative of formula (I) according to the claim 4 or non-toxic salts thereof as active ingredients.

7. A benzoic acid derivative of formula (I-A) according to claim 4 or non-toxic salts thereof for use in the prevention and / or treatment of bone diseases such as osteoporosis, rheumatoid arthritis, osteoarthritis, abnormal bone formation; cancer such as formation of cancer, proliferation of cancer, metastasis of cancer to organs and to bones and hypercalcemia induced metastasis to bones of cancer; systemic granuloma, immunological diseases such as ALS, multiple sclerosis, Sjoegren's syndrome, systemic lupus erythematosus, AIDS; allergy such as conjunctivitis, rhinitis, contact dermatitis, psoriasis; atopic dermatitis, asthma, pyorrhea, gingivitis, periodontitis, neuronal cell death, Alzheimer ' s disease's disease, pulmonary injury, hepatopathy, acute hepatopathy, nephritis, renal failure, myocardial ischemia, Kawasaki disease, scald, ulcerative colitis, Crohn's disease, multiple organ failure, sleeping disorder, platelet aggregation.
